(19) Europäisches Patentamt / European Patent Office / Office européen des brevets

(11) **EP 4 548 853 B1**

(12) **EUROPEAN PATENT SPECIFICATION**

(45) Date of publication and mention
of the grant of the patent:
**08.07.2026   Bulletin 2026/28**

(21) Application number: **24210838.9**

(22) Date of filing: **05.11.2024**

(51) International Patent Classification (IPC):
***A61B 6/46*** (2024.01)

(52) Cooperative Patent Classification (CPC):
**A61B 6/488; A61B 6/0492; A61B 6/08;
A61B 6/469; A61B 6/545;** A61B 6/032

(54) **INFORMATION PROCESSING APPARATUS, INFORMATION PROCESSING METHOD, AND INFORMATION PROCESSING PROGRAM**

INFORMATIONSVERARBEITUNGSVORRICHTUNG,
INFORMATIONSVERARBEITUNGSVERFAHREN UND
INFORMATIONSVERARBEITUNGSPROGRAMM

APPAREIL, PROCÉDÉ ET PROGRAMME DE TRAITEMENT D'INFORMATIONS

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:   **06.11.2023   JP 2023189613**

(43) Date of publication of application:
**07.05.2025   Bulletin 2025/19**

(73) Proprietor: **FUJIFILM Corporation
Tokyo 106-8620 (JP)**

(72) Inventor: **HAYASHI, Tetsuya
Tokyo, 106-8620 (JP)**

(74) Representative: **Dehns Germany Partnerschaft
mbB
Theresienstraße 6-8
80333 München (DE)**

(56) References cited:
**CN-A- 104 257 396     US-A1- 2017 224 298**

# Description

## BACKGROUND OF THE INVENTION

### Technical Field

The present invention relates to an information processing apparatus, an information processing method, and an information processing program.

### Related Art

In recent years, with the advancement of medical equipment, such as a computed tomography (CT) apparatus and a magnetic resonane imaging (MRI) apparatus, three-dimensional images having a higher-quality and a higher-resolution have been used for image diagnosis.

In a case in which imaging of a subject is performed with an imaging apparatus, such as the CT apparatus or the MRI apparatus, in order to determine an imaging range, scout imaging is performed before main imaging for acquiring a three-dimensional image to acquire a two-dimensional image for positioning (scout image). An operator (technician or the like) of the imaging apparatus sets an imaging range during the main imaging while viewing the scout image.

Before the scout imaging, the operator sets the imaging range of the scout imaging in the subject on an examination table. For example, the subject is irradiated with a cross-shaped laser, a scan start position of the scout imaging is set, and a scan end position of the scout imaging is set such that the imaging range corresponding to an imaging part is obtained. In a case of the scout imaging, the scan in the scout imaging is started in a case in which the examination table is moved from an initial position of the examination table to the scan start position, and the scan in the scout imaging ends in a case in which the examination table is moved to the scan end position. The operator sets the imaging range of the main imaging by using the scout image acquired by the scout imaging, and then performs the main imaging to acquire the three-dimensional image.

Here, during setting the imaging range during the scout imaging, the subject is imaged by a camera provided above the examination table, feature points such as both ankles, both waists, both elbows, and both shoulders of the subject are detected, and the imaging range is specified based on the detected feature points. In this case, a trained detection model constructed by training a neural network through machine learning is used for the detection of the feature points.

Meanwhile, a method has been proposed in which, in a case in which a trained model is applied to a medical image, trained models that performs a plurality of different types of processing is prepared, and a trained model to be used is selected depending on a situation, thereby efficiently processing the medical image (see, for example, JP2021-079013A). Further relevant prior art systems and methods are disclosed in the patent publications US 2017/224298 A1 and CN 104 257 396 A.

Meanwhile, the imaging apparatus is installed in the examination room, but in a case in which the lightness of the examination room is insufficient, the noise of the image acquired by the camera is increased, so that the feature points cannot be accurately detected. In a case in which the feature points cannot be accurately detected, the imaging range cannot be accurately specified.

## SUMMARY OF THE INVENTION

The present invention has been made in view of the above-described circumstances, and an object of the present invention is to enable appropriate detection of feature points in accordance with lightness of an examination room in a case of setting an imaging range.

The present invention relates to an information processing apparatus comprising: at least one processor, in which the processor acquires at least one of a first camera image generated by capturing a moving image of a subject on an examination table via a first camera or a second camera image generated by capturing a moving image of the subject via a second camera having higher imaging sensitivity than the first camera, selects at least one detection model from among a plurality of detection models including a first detection model constructed to detect a plurality of feature points on the subject included in the first camera image, and a second detection model constructed to detect the plurality of feature points on the subject included in the second camera image, detects the plurality of feature points on the subject included in the first camera image or in the second camera image by using the selected detection model, and specifies an imaging range of the subject based on the plurality of feature points. When this disclosure describes a device (e.g., the processor) performing a step, it especially includes the device being configured to perform the step.

The term "high imaging sensitivity" means that the imaging performance in a dark place is high. Therefore, as the second camera, for example, an NIR camera, a dark vision camera, or a camera having higher ISO sensitivity than the first camera is used.

It should be noted that, in the information processing apparatus according to an embodiment of the present invention, the processor may select the first detection model to detect the plurality of feature points on the subject included in the first camera image in a case in which lightness of an environment in which the examination table is installed is equal to or higher than a reference, and select the second detection model to detect the plurality of feature points on the subject included in the second camera image in a case in which the lightness is lower than the reference.

In addition, in the information processing apparatus according to an embodiment of the present invention, the processor may acquire the first camera image and determines the lightness based on brightness information derived from the first camera image.

In addition, in the information processing apparatus according to an embodiment of the present invention, the processor may acquire the first camera image and determines the lightness based on noise included in the first camera image.

In addition, in the information processing apparatus according to an embodiment of the present invention, the processor may acquire the first camera image, detect the feature points from the first camera image by using the first detection model, and determine the lightness based on detection accuracy of the feature points.

In addition, in the information processing apparatus according to an embodiment of the present invention, the processor may determine the lightness by using a sensor that detects the lightness of the environment.

In addition, in the information processing apparatus according to an embodiment of the present invention, the processor may acquire the first camera image, selects the first detection model to detect the feature points from the first camera image, determine whether or not the lightness is equal to or higher than the reference based on the first camera image, specify the imaging range based on the feature points detected by using the first detection model in a case in which the lightness is equal to or higher than the reference, select the second detection model in a case in which the lightness is lower than the reference, and specify the imaging range based on the feature points detected by using the second detection model.

In addition, in the information processing apparatus according to an embodiment of the present invention, the first detection model and the second detection model may be models that place importance on a frame rate in a case of detecting the feature points, the plurality of detection models may further include a third detection model that is constructed to detect the plurality of feature points on the subject included in the first camera image and that places importance on accuracy in a case of detecting the feature points, and a fourth detection model that is constructed to detect the plurality of feature points on the subject included in the second camera image and that places importance on accuracy in a case of detecting the feature points, and the processor may select the detection model in accordance with lightness of an environment in which the examination table is installed and an imaging part of the subject.

In addition, in the information processing apparatus according to an embodiment of the present invention, the processor may select any one of the first detection model or the third detection model to detect the plurality of feature points on the subject included in the first camera image in a case in which the lightness is equal to or higher than a reference, and select any one of the second detection model or the fourth detection model to detect the plurality of feature points on the subject included in the second camera image in a case in which the lightness is lower than the reference.

In addition, in the information processing apparatus according to an embodiment of the present invention, the processor may select any one of the first detection model or the third detection model and any one of the second detection model or the fourth detection model, in accordance with the imaging part of the subject.

In addition, in the information processing apparatus according to an embodiment of the present invention, the processor may determine detection accuracy of the feature points, specify the imaging range based on the feature points in a case in which the detection accuracy is equal to or higher than a reference, and issue an alert in a case in which the detection accuracy is lower than the reference.

In addition, in the information processing apparatus according to an embodiment of the present invention, the first detection model may be a model that places importance on a frame rate in a case of detecting the feature points, the plurality of detection models may further include a third detection model that is constructed to detect the plurality of feature points on the subject included in the first camera image and that places importance on accuracy in a case of detecting the feature points, and the processor may acquire the first camera image and the second camera image and selects the first detection model to detect the feature points from the first camera image, determine detection accuracy of the feature points detected by using the first detection model, specify the imaging range based on the feature points detected by using the first detection model in a case in which the detection accuracy is equal to or higher than a first reference, select the third detection model to detect the feature points from the first camera image by using the third detection model in a case in which the detection accuracy is lower than the first reference, determine detection accuracy of the feature points detected by using the third detection model, specify the imaging range based on the feature points detected by using the third detection model in a case in which the detection accuracy is equal to or higher than a second reference, select the second detection model to detect the feature points from the second camera image by using the second detection model in a case in which the detection accuracy is lower than the second reference, and specify the imaging range based on the feature points detected by using the second detection model.

In addition, in the information processing apparatus according to an embodiment of the present invention, the processor may determine the detection accuracy of the feature points detected by using the first detection model in a case in which the detection accuracy is equal to or higher than the first reference, the detection accuracy of the feature points detected by using the third detection model in a case in which the detection accuracy is equal to or higher than the second reference, or detection accuracy of the feature points detected by using the second detection model, specify, in a case in which the detection accuracy is equal to or higher than a third reference, the imaging range based on the feature points detected by using the first detection model in a case in which the detection accuracy is equal to or higher than the first reference, the feature points detected by using the third detection model in a case in which the detection accuracy is equal to or higher than the second reference, or the feature points detected by using the second detection model, and issue an alert in a case in which the detection accuracy is lower than the third reference.

In addition, in the information processing apparatus according to an embodiment of the present invention, the first detection model may be a model that places importance on a frame rate in a case of detecting the feature points, the plurality of detection models may further include a third detection model that is constructed to detect the plurality of feature points on the subject included in the first camera image and that places importance on accuracy in a case of detecting the feature points, and the processor may acquire the first camera image and the second camera image and selects the first detection model to detect the feature points from the first camera image, detect a movement of the subject based on the first camera image, specify the imaging range based on the feature points detected by using the first detection model in a case in which the movement of the subject is equal to or larger than a first reference, select the second detection model and the third detection model in a case in which the movement of the subject is smaller than the first reference, detect the feature points from the first camera image by using the third detection model, detect the feature points from the second camera image by using the second detection model, compare detection accuracy of the feature points detected by using the third detection model with detection accuracy of the feature points detected by using the second detection model, specifies the imaging range based on the feature points detected by using the third detection model in a case in which the detection accuracy of the feature points detected by using the third detection model is higher, and specify the imaging range based on the feature points detected by using the second detection model in a case in which the detection accuracy of the feature points detected by using the second detection model is higher.

In addition, in the information processing apparatus according to an embodiment of the present invention, the processor may determine detection accuracy of the feature points detected by using the first detection model in a case in which the movement is equal to or larger than the first reference, the detection accuracy of the feature points detected by using the third detection model, or the detection accuracy of the feature points detected by using the second detection model, specify, in a case in which the detection accuracy is equal to or higher than a second reference, the imaging range based on the feature points detected by using the first detection model in a case in which the movement is equal to or larger than the first reference, the feature points detected by using the third detection model, or the feature points detected by using the second detection model, and issue an alert in a case in which the detection accuracy is lower than the second reference.

In addition, in the information processing apparatus according to an embodiment of the present invention, the processor may derive a movement range of the examination table based on the imaging range.

In addition, in the information processing apparatus according to an embodiment of the present invention, the processor may display, on a display, a human body image imitating a human body and draws, on the human body image, a movement start line and a movement end line of the examination table based on the movement range of the examination table.

In addition, in the information processing apparatus according to an embodiment of the present invention, the imaging range may be an imaging range in a case of capturing an image for positioning acquired before main imaging of the subject is performed.

The present invention relates to an information processing method comprising: via a computer, acquiring at least one of a first camera image generated by capturing a moving image of a subject on an examination table via a first camera or a second camera image generated by capturing a moving image of the subject via a second camera having higher imaging sensitivity than the first camera; selecting at least one detection model from among a plurality of detection models including a first detection model constructed to detect a plurality of feature points on the subject included in the first camera image, and a second detection model constructed to detect the plurality of feature points on the subject included in the second camera image; detecting the plurality of feature points on the subject included in the first camera image or in the second camera image by using the selected detection model; and specifying an imaging range of the subject based on the plurality of feature points.

The present invention relates to an information processing program causing a computer to execute: a procedure of acquiring at least one of a first camera image generated by capturing a moving image of a subject on an examination table via a first camera or a second camera image generated by capturing a moving image of the subject via a second camera having higher imaging sensitivity than the first camera; a procedure of selecting at least one detection model from among a plurality of detection models including a first detection model constructed to detect a plurality of feature points on the subject included in the first camera image, and a second detection model constructed to detect the plurality of feature points on the subject included in the second camera image; a procedure of detecting the plurality of feature points on the subject included in the first camera image or in the second camera image by using the selected detection model; and a procedure of specifying an imaging range of the subject based on the plurality of feature points.

According to an embodiment of the present invention, the feature points can be appropriately detected in accordance with the lightness of the examination room in a case of setting the imaging range.

## BRIEF DESCRIPTION OF THE DRAWINGS

Fig. 1 is a perspective view showing an outline of a CT apparatus to which an information processing apparatus according to a first embodiment of the present invention is applied.

Fig. 2 is a side view showing the CT apparatus to which the information processing apparatus according to the first embodiment is applied.

Fig. 3 is a schematic perspective view showing an appearance of a camera.

Fig. 4 is a view showing a schematic configuration of the information processing apparatus according to the first embodiment.

Fig. 5 is a view showing a functional configuration of an information processing apparatus according to the first embodiment.

Fig. 6 is a view showing feature points.

Fig. 7 is a view showing a schema in which a scan start line and a scan end line are displayed.

Fig. 8 is a flowchart showing processing performed in the first embodiment.

Fig. 9 is a flowchart showing processing performed in a second embodiment.

Fig. 10 is a view showing a functional configuration of an information processing apparatus according to a third embodiment.

Fig. 11 is a flowchart showing processing performed in the third embodiment.

Fig. 12 is a flowchart showing processing performed in a fourth embodiment.

Fig. 13 is a flowchart showing the processing performed in the fourth embodiment.

Fig. 14 is a view showing a functional configuration of an information processing apparatus according to a fifth embodiment.

Fig. 15 is a flowchart showing processing performed in the fifth embodiment.

Fig. 16 is a flowchart showing the processing performed in the fifth embodiment.

## DETAILED DESCRIPTION

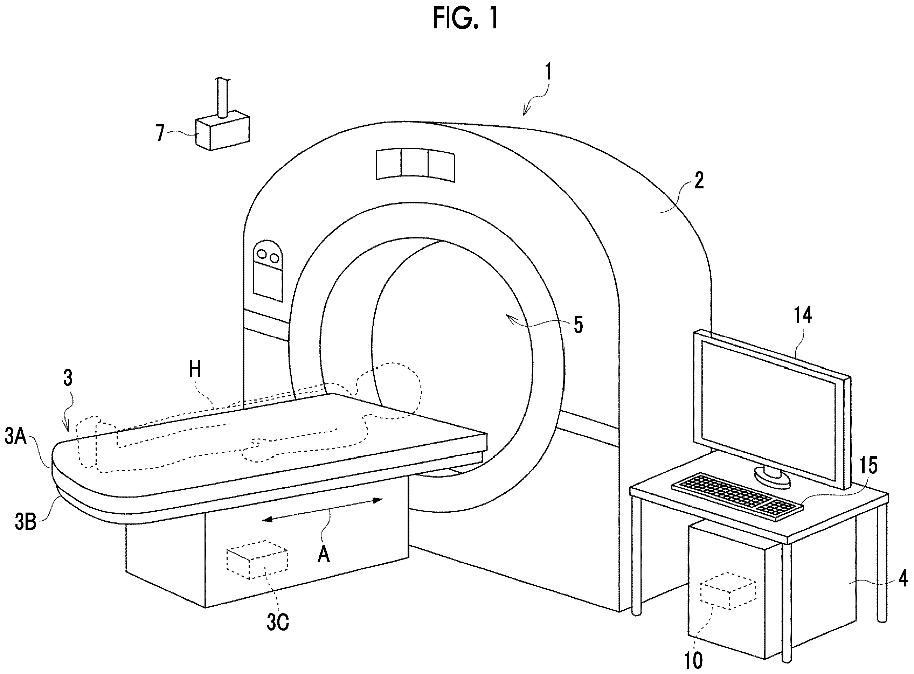

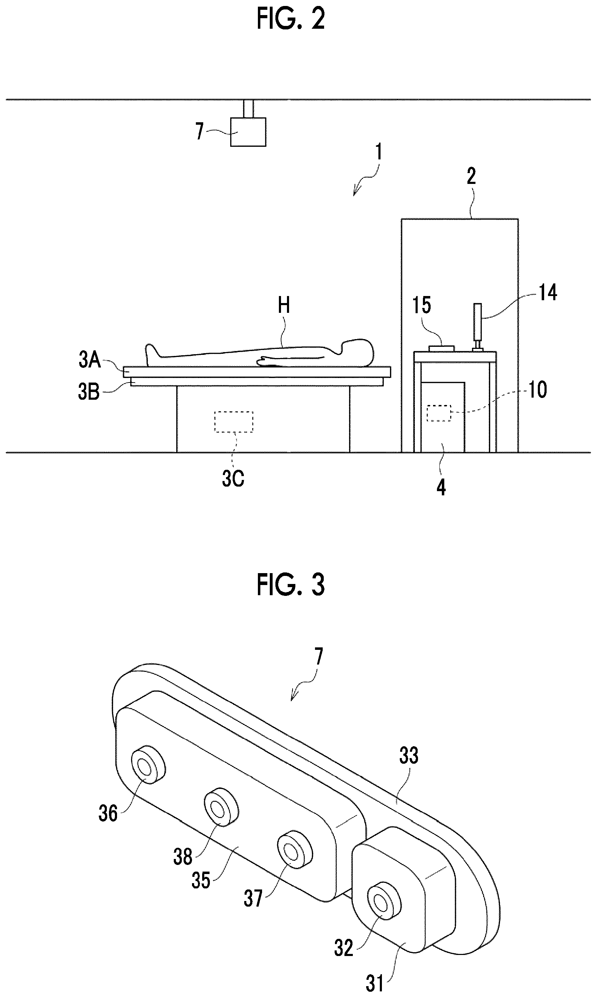

Hereinafter, description regarding embodiments of the present invention will be made with reference to the drawings. Fig. 1 is a perspective view showing an outline of a CT apparatus to which an information processing apparatus according to a first embodiment of the present invention is applied, and Fig. 2 is a side view showing the CT apparatus to which the information processing apparatus according to the first embodiment of the present invention is applied. As shown in Figs. 1 and 2, the CT apparatus 1 according to an embodiment of the present embodiment comprises a gantry 2, an examination table 3, and a console 4.

The gantry 2 has a tunnel-shaped structure with an opening portion 5 at the center thereof. Inside the gantry 2, a radiation source unit that emits X-rays and a detection unit that detects the X-rays to generate a radiation image are provided (neither of which is shown). The radiation source unit and the detection unit can rotate along an annular shape of the gantry 2 in a state in which a positional relationship in which the radiation source unit and the detection unit face each other is maintained. A controller that controls an operation of the CT apparatus 1 is provided inside the gantry 2.

The examination table 3 includes an examination table part 3A on which an subject lies down, a base part 3B that supports the examination table part 3A, and a driving part 3C that reciprocally moves the examination table part 3A in an arrow A direction. The examination table part 3A is slidable with respect to the base part 3B in the arrow A direction via the driving part 3C. In a case in which a CT image is captured, the examination table part 3A is slid, and a subject H lying down on the examination table part 3A is transported into the opening portion 5 of the gantry 2.

It should be noted that a camera 7 is installed above the examination table 3. The camera 7 is configured by integrating an RGB camera that can capture an RGB color image by detecting reflected light of the subject H and a near infrared (NIR) camera that can perform stereo imaging. Fig. 3 is a schematic perspective view showing an appearance of the camera 7. As shown in Fig. 3, the camera 7 is configured by attaching an RGB camera 31 and an NIR camera 35 to the base part 33. The RGB camera 31 includes an RGB sensor 32 including a lens and an imaging element such as a charge coupled device (CCD). The RGB camera 31 acquires an RGB camera image, which is a RGB color moving image, by imaging the subject H on the examination table 3 at a predetermined frame rate, and outputs the RGB camera image to the console 4.

The NIR camera 35 includes a left NIR sensor 36 and a right NIR sensor 37, which include the imaging element such as the lens and the CCD, and an NIR projector 38. The NIR camera 35 emits near-infrared rays from the NIR projector 38 toward the subject H, and the left and right NIR sensors 36 and 37 detect reflected light of the near-infrared rays from the subject H at a predetermined frame rate. As a result, the NIR camera 35 acquires left and right NIR camera images, and outputs the acquired left and right NIR camera images to the console 4. It should be noted that the left and right NIR camera images are monochrome images. Here, since the left NIR sensor 36 and the right NIR sensor 37 are separated from each other, the left and right NIR camera images have parallax. Therefore, the camera 7 derives depth information of the subject H included in the left and right NIR camera images and the other subjects based on the parallax, and outputs the depth information along with the left and right NIR camera images. It should be noted that, in the following description, in a case in which the term "NIR camera image" is simply used, the NIR camera image refers to any one of the left NIR camera image or the right NIR camera image.

Since the NIR camera 35 performs the imaging based on the near-infrared rays, even in a case in which an imaging room in which the CT apparatus 1 is installed is dark, the NIR camera 35 can acquire the NIR camera image in which the subject H can be visually recognized. On the other hand, since the RGB camera 31 performs the imaging based on visible light, in a case in which lightness of an environment is insufficient, it is difficult to visually recognize the subject H in the acquired RGB camera image. Therefore, in order to acquire the RGB camera image in which the subject H can be visually recognized, the imaging room needs to be bright to some extent. The RGB camera 31 is an example of a first camera of the present invention. The NIR camera 35 is an example of a second camera having higher imaging sensitivity than the first camera of the present invention. The RGB camera image is an example of a first camera image of the present invention, and the NIR camera image is an example of a second camera image of the present invention.

It should be noted that, in the present embodiment, the camera 7 simultaneously acquires the RGB camera image acquired by the RGB camera 31 and the NIR camera image acquired by the NIR camera 35.

The driving of the gantry 2, the driving of the examination table 3, and the imaging of the subject H via the camera 7 are performed in response to an input from the operator through the console 4. The console 4 includes the information processing apparatus according to the first embodiment.

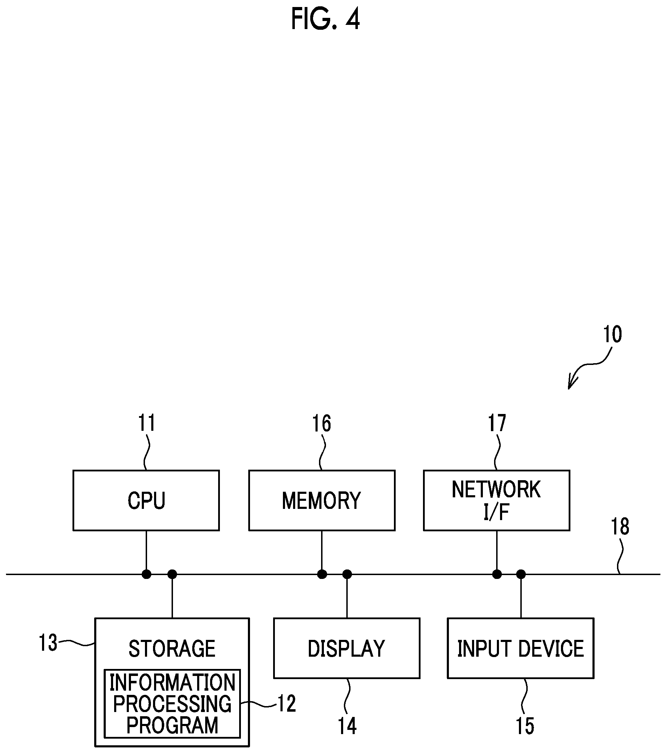

Next, description regarding the information processing apparatus according to the first embodiment, which is included in the console 4 will be made. First, a hardware configuration of the information processing apparatus according to the first embodiment will be described with reference to Fig. 4. As shown in Fig. 4, an information processing apparatus 10 is a computer, such as a workstation, a server computer, and a personal computer, and comprises a central processing unit (CPU) 11, a non-volatile storage 13, and a memory 16 as a transitory storage area. The information processing apparatus 10 further comprises a display 14 such as a liquid crystal display, an input device 15 such as a keyboard and a mouse, and an interface such as a network interface (I/F) 17 connected to the CT apparatus 1. The CPU 11, the storage 13, the display 14, the input device 15, the memory 16, and the network I/F 17 are connected to a bus 18. It should be noted that the CPU 11 is an example of a processor of the present invention. The display 14 and the input device 15 are also shown in Figs.

1 and 2.

The storage 13 is realized by a hard disk drive (HDD), a solid state drive (SSD), a flash memory, and the like. The storage 13 as a storage medium stores an information processing program 12 installed in the information processing apparatus 10. The CPU 11 reads out the information processing program 12 from the storage 13, loads the readout information processing program 12 into the memory 16, and executes the loaded information processing program 12.

It should be noted that the information processing program 12 is stored in a storage device of a server computer connected to a network or in a network storage in a state of being accessible from the outside, and is downloaded and installed in the computer constituting the information processing apparatus 10 in response to a request. Alternatively, the information processing program 12 is distributed in a state of being recorded on a recording medium, such as a digital versatile disc (DVD) or a compact disc read only memory (CD-ROM), and is installed in the computer constituting the information processing apparatus 10 from the recording medium.

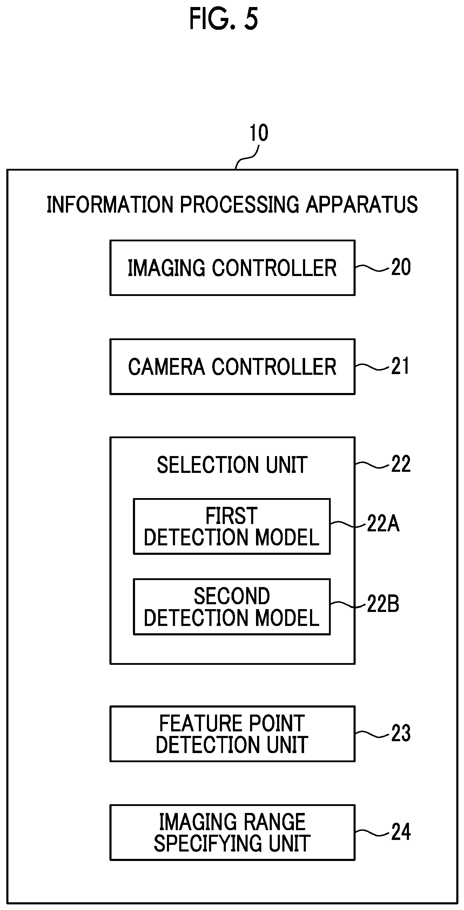

Next, description regarding a functional configuration of the information processing apparatus according to the first embodiment will be made. Fig. 5 is a view showing the functional configuration of the information processing apparatus according to the first embodiment. As shown in Fig. 5, the information processing apparatus 10 comprises an imaging controller 20, a camera controller 21, a selection unit 22, a feature point detection unit 23, and an imaging range specifying unit 24. The CPU 11 executes the information processing program 12, whereby the CPU 11 functions as the imaging controller 20, the camera controller 21, the selection unit 22, the feature point detection unit 23, and the imaging range specifying unit 24.

The imaging controller 20 controls an imaging unit, the detection unit, and the controller provided in the gantry 2 to perform the imaging of the subject H in response to an instruction from the input device 15. It should be noted that, in a case of CT imaging, scout imaging is performed before main imaging for acquiring a three-dimensional CT image in order to determine an imaging range. The scout imaging is performed by imaging the subject H, with the imaging unit and the detection unit being in a fixed state.

In a case of the scout imaging, as will be described below, the imaging range of the subject H is set, and the set imaging range is imaged by moving the examination table 3 to the opening portion 5 of the gantry 2 to perform the scout imaging. An scout image acquired by the scout imaging is a two-dimensional X-ray image including the imaging range set for the subject H. The display 14 displays the scout image. The operator views the scout image displayed on the display 14, thereby setting the imaging range in a case of performing the main imaging. After the imaging range is set, the operator inputs an instruction for the main imaging from the input device 15, so that the main imaging is performed and the three-dimensional CT image of the subject H is acquired. The storage 13 stores the acquired scout image and CT image.

The camera controller 21 controls the imaging of the subject H on the examination table 3 via the camera 7. The imaging of the subject H via the camera 7 is performed to set the imaging range in a case of performing the scout imaging. The imaging of the subject H via the camera 7 is performed from a preparation stage before the imaging. That is, the camera controller 21 starts the imaging via the camera 7 from a point in time before the subject H lies down on the examination table 3 in a supine position, and causes the camera 7 to acquire the camera image. Then, in a case in which the operator issues an instruction to start the scout imaging, the camera controller 21 stops the imaging via the camera 7. The acquired camera image is stored in the memory 16 in order to specify the imaging range described below. In the following description, the RGB camera image and the NIR camera image may be simply referred to as the camera image.

In the first embodiment, the selection unit 22 selects one detection model from a first detection model 22A constructed to detect a plurality of feature points on the subject H included in the RGB camera image acquired by the camera 7 and a second detection model 22B constructed to detect the plurality of feature points on the subject H included in the NIR camera image. The first detection model 22A and the second detection model 22B are constructed by training a neural network through machine learning.

Since the first detection model 22A detects the feature points from the RGB camera image, a model constructed to detect the feature points from the color image is used. Since the second detection model 22B detects the feature points from the NIR camera image, a model constructed to detect the feature points from the monochrome image is used.

In the training of the first detection model 22A, a color image in which an entire human body is included and 17 feature points are known is used as a training image. Specifically, the RGB camera image acquired by the RGB camera 31 is used. In the training of the second detection model 22B, a monochrome image in which the entire human body is included and 17 feature points are known is used as a training image. In practice, any one of the left NIR camera image or the right NIR camera image acquired by the NIR camera 35 is used as the training image. It should be noted that the training image is acquired by, for example, imaging a person wearing an examination gown via the camera 7 in the same manner as in a case of actually performing an examination.

It should be noted that the neural network having the same structure may be used for the first detection model 22A and the second detection model 22B, or neural networks having structures suitable for the detection of the feature points from the RGB camera image and the NIR camera image, respectively, may be used.

In the first embodiment, the selection unit 22 selects any one of the first detection model 22A or the second detection model 22B in accordance with the lightness of the imaging room in which the examination table 3 is installed. The lightness may be determined based on, for example, brightness information derived from the RGB camera image. In this case, the selection unit 22 may derive the brightness of each pixel of the RGB camera image, derive an average value of the brightness of all the pixels of the RGB camera image as the brightness information, make a determination as being bright in a case in which the brightness information is equal to or higher than a threshold value, and make a determination as being dark in a case in which the brightness information is lower than the threshold value. It should be noted that the brightness of each pixel can be derived by Expression (1) based on signal values of R, G, and B of the respective pixels. In Expression (1), Y represents the brightness.

$$Y = 0.299 \times R + 0.587 \times G + 0.114 \times B \ (1)$$

Meanwhile, in a case in which the lightness is insufficient, the RGB camera image includes a large amount of noise. Therefore, the selection unit 22 may derive noise characteristics of the RGB camera image and determine the lightness based on the noise characteristics. As the noise characteristics, the standard deviation of the pixel values of the respective pixels of the RGB camera image can be used. The standard deviation need only be calculated by the root mean square (RMS) of the pixel values of the respective pixels of the RGB camera image. In this case, the selection unit 22 need only calculate the average value $N^2m$ of the squared pixel values of the respective pixels of the RGB camera image and then take the square root of the average value $N^2m$ (that is, $\sqrt{N^2m}$), thereby calculating the standard deviation. The selection unit 22 needs only to make a determination as being dark in a case in which the standard deviation is equal to or larger than a predetermined threshold value, and make a determination as being bright in a case in which the standard deviation is smaller than the threshold value. It should be noted that, instead of the standard deviation, the noise characteristics may be calculated as a variance of the pixel values of the RGB camera image or a difference between the maximum value and the minimum value.

In addition, a lightness sensor that detects the lightness in the vicinity of the examination table 3 may be installed in the CT apparatus 1 or in the imaging room, and the lightness may be determined based on the output of the lightness sensor. In this case, the selection unit 22 needs only to make a determination as being bright in a case in which a detection value of the lightness sensor is equal to or higher than a predetermined threshold value, and make a determination as being dark in a case in which the detection value of the lightness sensor is lower than the threshold value.

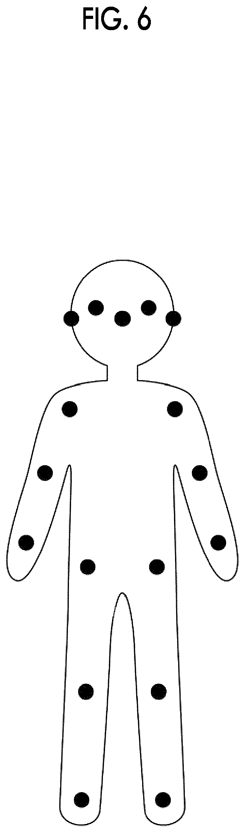

The feature point detection unit 23 detects the feature points from the camera image by using the detection model selected by the selection unit 22. Fig. 6 is a view showing the feature points. As shown in Fig. 6, in the present embodiment, the first and second detection models 22A and 22B are constructed to detect the 17 feature points on the subject H included in the camera image. The 17 feature points are both eyes, a nose, both ears, both shoulders, both elbows, both hands, both waists, both knees, and both feet. It should be noted that 18 feature points may be used by adding a point at the center of the clavicle to the 17 feature points.

The first detection model 22A and the second detection model 22B derive a probability representing a possibility of each of 17 feature points for each pixel of the camera image. The feature point detection unit 23 detects a pixel from which the highest probability is derived, as the feature point, for each of the 17 feature points. For example, in a case in which a right eye is detected as the feature point, the feature point detection unit 23 compares the probabilities of the right eye for all the pixels of the camera images derived by the first detection model 22A and the second detection model 22B, and detects the pixel having the highest probability as the feature point of the right eye.

The imaging range specifying unit 24 specifies the imaging range of the subject H in a case in which the scout imaging is performed based on the 17 feature points detected by using the feature point detection unit 23. Therefore, the imaging range specifying unit 24 first determines the detection accuracy of the feature points detected by using the feature point detection unit 23. As described above, the feature point detection unit 23 detects the pixel from which the highest probability is derived, as the feature point, for each of the 17 feature points. The detection accuracy is higher as the probability output by the detection model is higher for the detected feature point. Therefore, the imaging range specifying unit 24 compares a representative value of the probabilities derived by the detection model for the 17 feature points with a predetermined threshold value, determines that the detection accuracy is high in a case in which the representative value is equal to or higher than the threshold value, and determines that the detection accuracy is low in a case in which the representative value is lower than the threshold value. As the representative value, an average value, a median value, a weighted average value corresponding to the imaging part, or the like can be used, but the present invention is not limited thereto.

The imaging range specifying unit 24 performs processing of specifying the imaging range in a case in which it is determined that the detection accuracy is high. On the other hand, the imaging range specifying unit 24 performs alert display on the display 14 in a case in which it is determined that the detection accuracy is low. Here, in a case in which the subject H moves too much, a thick blanket is placed on the subject H, or the entire body of the subject H is not included in the imaging range of the camera 7, the feature points cannot be accurately detected regardless of which detection model is used, and the detection accuracy is deteriorated. In this case, the imaging range specifying unit 24 determines that the detection accuracy is low.

It should be noted that, in a case in which the alert display is performed, the operator manually sets the imaging range of the scout imaging. That is, the operator measures a distance from an initial position of the examination table to a scan start line, further measures a distance between the scan start line and a scan end line, and inputs the measured distances from the input device 15. The movement of the examination table 3 during the scout imaging is controlled based on the input distances.

Next, description regarding the processing of specifying the imaging range via the imaging range specifying unit 24 will be made. For example, in a case in which the imaging part is the head, the scout image is an image in which a range from the top of the head to the tip of the chin is the imaging range. Therefore, the imaging range specifying unit 24 sets a line connecting both eyes or both ears among the feature points detected by using the feature point detection unit 23, and further sets the scan start line at the top of the head and the scan end line between the chin and both shoulders. Then, a distance D1 between the scan start line and the scan end line is derived based on a relationship between the line connecting the both eyes or the both ears and the distance between the scan start line and the scan end line. A range of the distance D1 between the scan start line and the scan end line is the imaging range.

Here, before the scout imaging, the examination table 3 is at the initial position, and the subject H lies down on the examination table 3 in a supine position, so that a distance from an end part of the examination table 3 to the top of the head of the subject H can be known from the camera image. Therefore, the imaging range specifying unit 24 calculates a distance D2 from the end part of the examination table 3 to the top of the head of the subject H as a movement amount of the examination table 3 from the initial position to the scan start line, that is, a movement amount of the examination table 3 for the top of the head the subject H to reach the scan position in the CT apparatus 1.

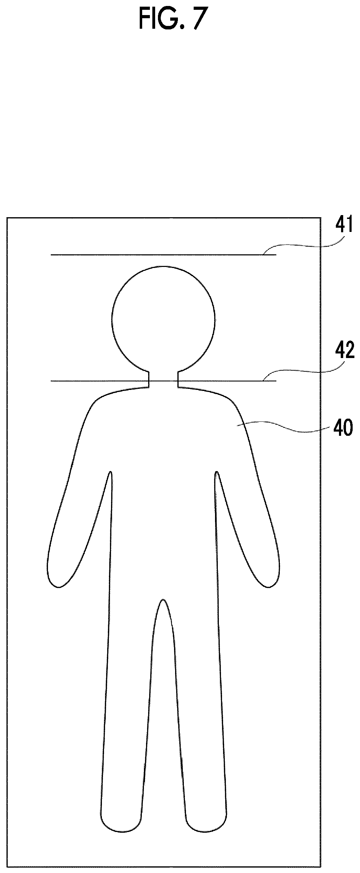

In a case in which the imaging range is specified, the imaging range specifying unit 24 displays the scan start line and the scan end line on a schema that is a human body diagram displayed on the display 14. Fig. 7 is a view showing a schema in which the start line and the end line are displayed. As shown in Fig. 7, a scan start line 41 and a scan end line 42 are shown in a schema 40. The operator checks the scan start line and the scan end line displayed on the display 14. After the check, in a case of OK, the operator issues the instruction to start the scout imaging by using the input device 15.

The information on the distances D1 and D2 is output to the CT apparatus 1 in response to the instruction to start the scout imaging. The imaging controller 20 controls the CT apparatus 1 such that the scan in the scout imaging is started after the driving part 3C moves the examination table 3 by the distance D2, and the scan in the scout imaging ends in a case in which the driving part 3C moves the examination table 3 by the distance D1.

The scout image acquired by the scout imaging is displayed on the display 14. The operator checks the scout image displayed on the display 14, and sets the imaging range of the main imaging on the scout image. Thereafter, the main imaging is performed by issuing an imaging instruction for the main imaging by using the input device 15, and the three-dimensional CT image of the imaging part of the subject H is acquired in the set imaging range of the main imaging.

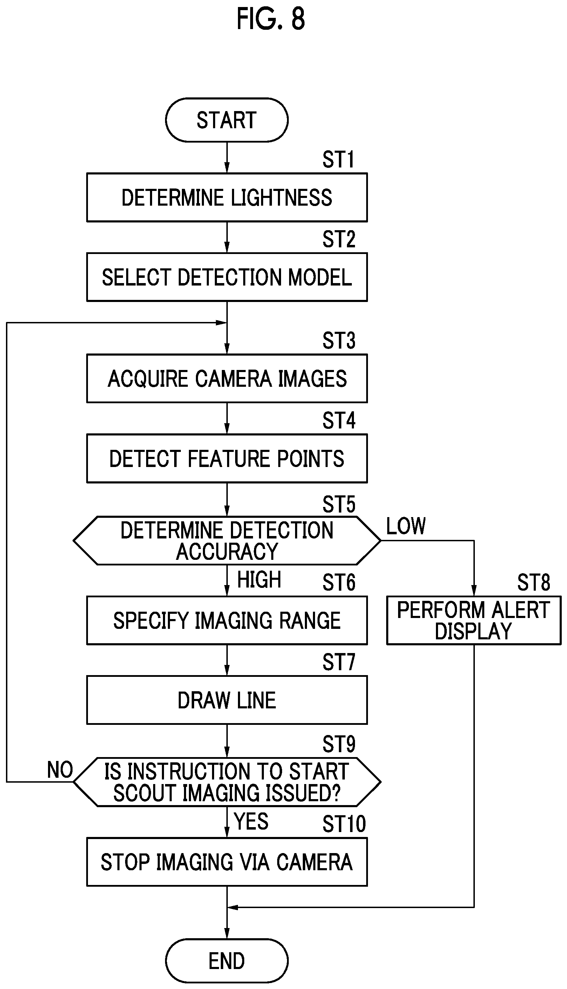

Next, description regarding processing performed in the first embodiment will be made. Fig. 8 is a flowchart showing the processing performed in the first embodiment. The processing is started in a case in which the imaging start instruction is issued from the input device 15, and the selection unit 22 determines the lightness of the imaging room in which the CT apparatus 1 is installed (step ST1). Then, the selection unit 22 selects any one of the first detection model 22A or the second detection model 22B in accordance with the determined lightness (detection model selection; step ST2).

Then, the camera controller 21 starts the imaging via the camera 7 to acquire the camera image (step ST3), and the feature point detection unit 23 detects the feature points from the camera image by using the detection model selected by the selection unit 22 (step ST4). Then, the imaging range specifying unit 24 determines the detection accuracy of the feature points (step ST5). The imaging range specifying unit 24 specifies the imaging range during the scout imaging based on the feature points in a case in which it is determined that the detection accuracy is high (step ST6), and draws the scan start line and the scan end line during the scout imaging on the schema displayed on the display 14 based on the specified imaging range (line drawing; step ST7). On the other hand, in a case in which it is determined that the detection accuracy is low, the imaging range specifying unit 24 performs the alert display (step ST8). In a case in which the alert display is performed, the information processing apparatus 10 ends the imaging range specifying processing. In this case, the operator manually sets the imaging range of the scout imaging as described above.

Then, it is determined whether or not the instruction to start the scout imaging is issued by the operator (step ST9), the processing returns to step ST3 in a case in which a negative determination is made in step ST9, and the processing in and after step ST3 is repeated. In a case in which an affirmative determination is made in step ST9, the camera controller 21 stops the imaging via the camera 7 (step ST10), and the information processing apparatus 10 ends the imaging range specifying processing.

Thereafter, the scout image is acquired by performing the scout imaging via the imaging controller 20, and is displayed on the display 14. The operator checks the scout image, and then sets the imaging range of the main imaging. Then, the operator issues the instruction for the main imaging from the input device 15 to perform the main imaging, and the three-dimensional CT image of the subject H is acquired.

As described above, in the first embodiment, the detection model used for detecting the feature points is selected in accordance with the lightness of the examination room. Therefore, the first detection model 22A that detects the feature points from the RGB camera image can be selected in a case in which the examination room is bright, and the second detection model 22B that detects the feature points from the NIR camera image can be selected in a case in which the examination room is dark. Therefore, in a case of setting the imaging range, the feature points can be appropriately detected in accordance with the lightness of the examination room, and as a result, the imaging range during the scout imaging can be appropriately specified by using the detected feature points.

It should be noted that, in the first embodiment, both the RGB camera image and the NIR camera image are acquired by the camera 7, but the present invention is not limited thereto. Only the RGB camera image may be acquired by the RGB camera 31 of the camera 7 in a case in which the selection unit 22 makes a determination as being bright, and only the NIR camera image may be acquired by the NIR camera 35 of the camera 7 in a case in which the selection unit 22 makes a determination as being dark.

Next, description regarding a second embodiment of the present invention will be made. It should be noted that, since a hardware configuration and a functional configuration of an information processing apparatus according to the second embodiment are the same as those of the information processing apparatus according to the first embodiment, the detailed description thereof will be omitted here. The second embodiment is different from the first embodiment in that the information processing apparatus 10 according to the second embodiment first selects the first detection model 22A to detect the feature points from the RGB camera image, determines whether or not the lightness is equal to or higher than a reference based on the RGB camera image, specifies the imaging range based on the feature points detected from the RGB camera image by using the first detection model 22A in a case in which the lightness is equal to or higher than the reference, and selects the second detection model 22B to specify the imaging range based on the feature points detected from the NIR camera image by using the second detection model 22B in a case in which the lightness is lower than the reference.

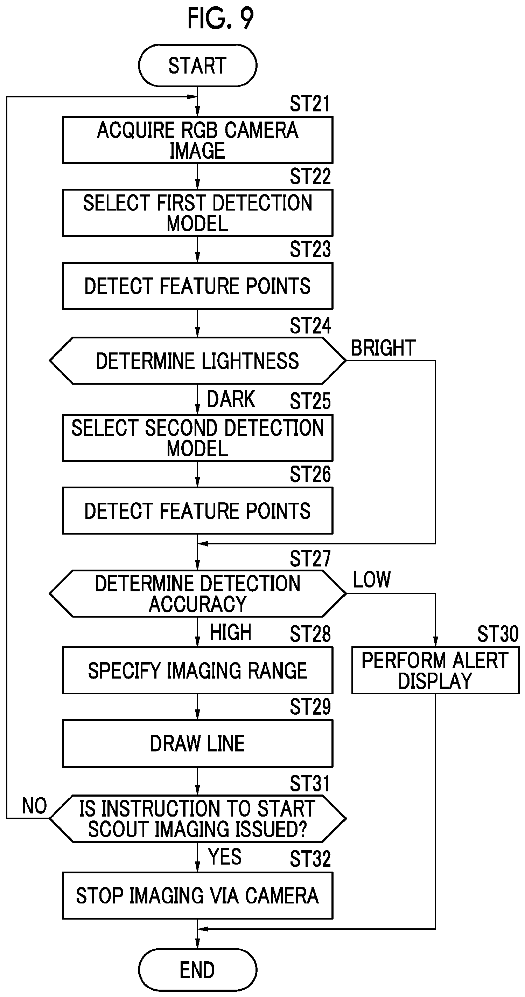

Next, description regarding processing performed in the second embodiment will be made. Fig. 9 is a flowchart showing the processing performed in the second embodiment. For example, the processing is started in response to the instruction to start the imaging from the input device 15, and the camera controller 21 starts the imaging via the camera 7 to acquire the RGB camera image (step ST21). Next, the selection unit 22 selects the first detection model 22A (step ST22), and the feature point detection unit 23 detects the feature points from the RGB camera image by using the first detection model 22A selected by the selection unit 22 (step ST23).

Then, the selection unit 22 derives the brightness information from the RGB camera image, and determines the lightness of the examination room in which the CT apparatus 1 is installed, based on the brightness information (step ST24). In a case in which a determination is made as being dark as the lightness, the selection unit 22 selects the second detection model 22B instead of the first detection model 22A (step ST25). Then, the feature point detection unit 23 detects the feature points from the NIR camera image by using the second detection model 22B (step ST26), and the imaging range specifying unit 24 determines the detection accuracy of the feature points (step ST27). In a case in which a determination is made as being bright in step ST24, the processing proceeds to step ST27, and the imaging range specifying unit 24 determines the detection accuracy of the feature points detected from the RGB camera image in step ST23.

The imaging range specifying unit 24 specifies the imaging range during the scout imaging based on the feature points in a case in which it is determined that the detection accuracy is high (step ST28), and draws the scan start line and the scan end line during the scout imaging on the schema displayed on the display 14 based on the specified imaging range (line drawing; step ST29). On the other hand, in a case in which it is determined that the detection accuracy is low, the imaging range specifying unit 24 performs the alert display (step ST30). In a case in which the alert display is performed, the information processing apparatus 10 ends the imaging range specifying processing. In this case, the operator manually sets the imaging range of the scout imaging as described above.

Then, it is determined whether or not the instruction to start the scout imaging is issued by the operator (step ST31), the processing returns to step ST21 in a case in which a negative determination is made in step ST31, and the processing in and after step ST21 is repeated. In a case in which an affirmative determination is made in step ST31, the camera controller 21 stops the imaging via the camera 7 (step ST32), and the information processing apparatus 10 ends the imaging range specifying processing.

As described above, in the second embodiment, the feature points are detected from the RGB camera image by using the first detection model 22A, the lightness is determined by using the RGB camera image, the detected feature points are used as they are in a case of being bright, and the feature points are detected from the NIR camera image by using the second detection model 22B in a case of being dark. Therefore, in a case of setting the imaging range, the feature points can be appropriately detected in accordance with the lightness of the imaging room, and as a result, the imaging range during the scout imaging can be appropriately specified by using the detected feature points.

It should be noted that, in the second embodiment, the lightness is determined based on the brightness information derived from the RGB camera image, but the present invention is not limited thereto. The feature points may be detected from the RGB camera image by using the first detection model 22A, and the lightness may be determined based on the detection accuracy of the feature points. Here, in a case in which the examination room is dark, it is difficult to visually recognize the subject H in the RGB camera image, so that the detection accuracy of the feature points is lowered. Therefore, the selection unit 22 may compare a representative value of the probabilities derived by the detection model for the 17 feature points with a predetermined threshold value, determine that the detection accuracy is high and thus the examination room is bright in a case in which the representative value is equal to or higher than the threshold value, and determine that the detection accuracy is low and thus the examination room is dark in a case in which the representative value is lower than the threshold value.

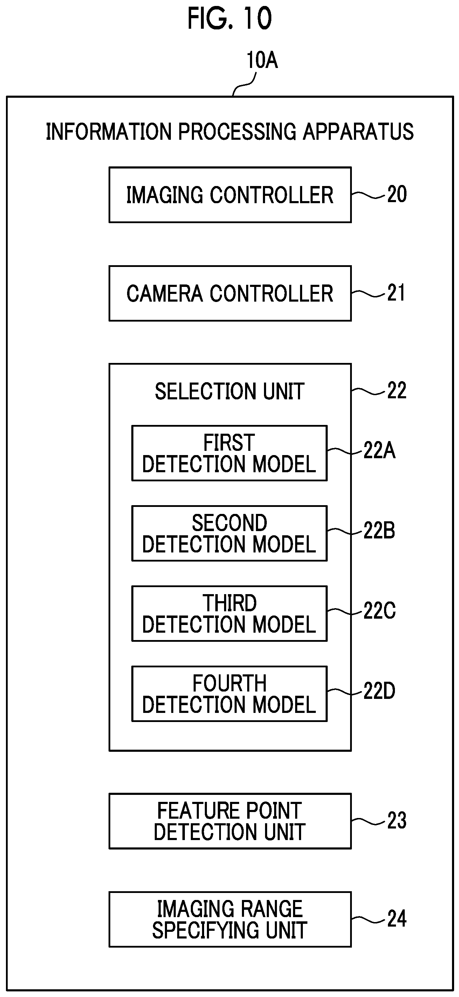

Next, description regarding a third embodiment of the present invention will be made. It should be noted that a hardware configuration of an information processing apparatus according to the third embodiment is the same as the hardware configuration of the information processing apparatus according to the first embodiment, and thus the detailed description thereof will be omitted here. Fig. 10 is a view showing a functional configuration of the information processing apparatus according to the third embodiment. In Fig. 10, the same reference numerals are applied to the same configurations as those in Fig. 5, and the detailed description thereof will be omitted. The third embodiment is different from the first embodiment in that an information processing apparatus 10A according to the third embodiment includes a third detection model 22C and a fourth detection model 22D in addition to the first detection model 22A and the second detection model 22B, and selects the detection model used for detecting the feature points from among the first detection model 22A, the second detection model 22B, the third detection model 22C, and the fourth detection model 22D.

In the third embodiment, the first detection model 22A is a model that places importance on a frame rate in a case of detecting the feature points from the RGB camera image, and the second detection model 22B is a model that places importance on a frame rate in a case of detecting the feature points from the NIR camera image. The third detection model 22C is a model that places importance on the accuracy in a case of detecting the feature points from the RGB camera image, and the fourth detection model 22D is a model that places importance on the accuracy in a case of detecting the feature points from the NIR camera image.

The phrase "place importance on a frame rate" means that the processing speed for detecting the feature points is improved by, for example, reducing the amount of data to be processed, omitting the calculation, or the like. Since the first detection model 22A and the second detection model 22B place importance on the frame rate, a model having a small amount of calculation and a high processing speed for detecting the feature points is used.

The phrase "place importance on accuracy" means that the accuracy in a case of detecting the feature points is improved without reducing the amount of data or omitting the calculation, although the calculation takes time. Since the importance is placed on the accuracy, the third detection model 22C and the fourth detection model 22D have a large amount of calculation and has a lower processing speed than the first detection model 22A and the second detection model 22B, but a model having higher accuracy of detecting the feature points than the first detection model 22A and the second detection model 22B is used.

As the first detection model 22A and the second detection model 22B, a neural network having a structure capable of performing processing of detecting the feature points at high speed with a small amount of calculation is used. The third detection model 22C and the fourth detection model 22D use a neural network having a structure having a larger amount of calculation but capable of detecting the feature points with higher accuracy than the first detection model 22A and the second detection model 22B. For any neural network, a known training image in which the entire human body, which is acquired by imaging the human body via the camera 7, is included and the 17 feature points are known is used for learning. It should be noted that the training image is acquired by, for example, imaging a person wearing an examination gown via the camera 7 in the same manner as in a case of actually performing an examination. In the third embodiment, the RGB camera image is used as the training image for the training of the first detection model 22A and the third detection model 22C. In addition, the NIR camera image, which is a gray image, is used as the training image for the training of the second detection model 22B and the fourth detection model 22D.

It should be noted that the neural network having the same structure may be used for the first detection model 22A and the second detection model 22B, and the neural network having the same structure may be used for the third detection model 22C and the fourth detection model 22D. In this case, the neural network is trained using different training images between the first detection model 22A and the second detection model 22B, and the third detection model 22C and the fourth detection model 22D. For example, a first training image used for the training of the first detection model 22A and the second detection model 22B has a relatively low resolution. On the other hand, as a second training image used for the training of the third detection model 22C and the fourth detection model 22D, an image having a higher resolution than the first training image is used.

In the third embodiment, the selection unit 22 selects the detection model in accordance with the lightness of the examination room and the imaging part of the subject H. Here, the imaging parts of the subject H include a head, a chest, an abdomen, a lower limb, and the entire body, but the head is likely to move during the imaging, and the chest or the abdomen is less likely to move during the imaging. Therefore, in a case in which the examination room is bright and the imaging part is the head or the entire body including the head, the selection unit 22 selects the first detection model 22A that places importance on the frame rate in a case of detecting the feature points from the RGB camera image. On the other hand, in a case in which the examination room is dark, the imaging part is the head or the entire body including the head, the selection unit 22 selects the second detection model 22B that places importance on the frame rate in a case of detecting the feature points from the NIR camera image.

In a case in which the imaging part is an abdomen or a lower limb, the imaging is often performed by covering the abdomen or the lower limb with a blanket. In such a case, in a case in which the examination room is bright, the selection unit 22 selects the third detection model 22C that places importance on the accuracy in a case of detecting the feature points from the RGB camera image. In addition, in a case in which the examination room is dark, the selection unit 22 selects the fourth detection model 22D that places importance on the accuracy in a case of detecting the feature points from the NIR camera image.

It should be noted that which part of the subject H is imaged is included in an examination order provided by a doctor in a case of the imaging, and is set by the operator from the input device 15 in accordance with the examination order.

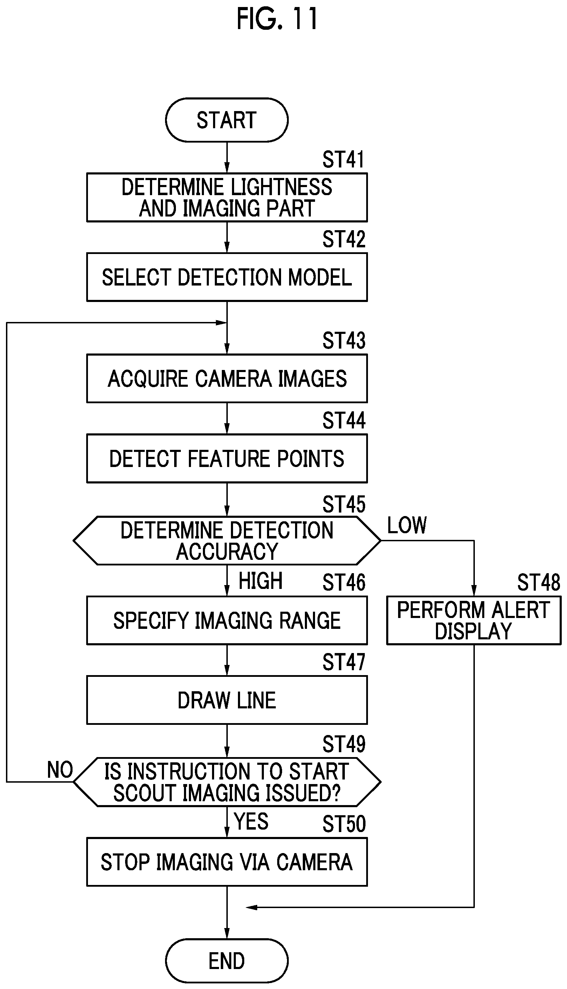

Next, description regarding processing performed in the third embodiment will be made. Fig. 11 is a flowchart showing processing performed in the third embodiment. For example, the processing is started in a case in which the imaging start instruction is issued from the input device 15, and the selection unit 22 determines the lightness of the imaging room in which the CT apparatus 1 is installed and the imaging part included in the examination order (step ST41). Then, the selection unit 22 selects any one of the first detection model 22A, the second detection model 22B, the third detection model 22C, or the fourth detection model 22D in accordance with the determined lightness (detection model selection; step ST42).

The selection unit 22 selects the first detection model 22A in a case in which a determination is made as being bright and the imaging part is likely to move, and selects the second detection model 22B in a case in which a determination is made as being dark and the imaging part is likely to move. The selection unit 22 selects the third detection model 22C in a case in which a determination is made as being bright and the imaging part is less likely to move, and selects the fourth detection model 22D in a case in which a determination is made as being dark and the imaging part is less likely to move.

Then, the camera controller 21 starts the imaging via the camera 7 to acquire the camera image (step ST43), and the feature point detection unit 23 detects the feature points from the camera image by using the detection model selected by the selection unit 22 (step ST44).

That is, in a case in which the first detection model 22A is selected, the feature point detection unit 23 detects the feature points from the RGB camera image by using the first detection model 22A. In a case in which the second detection model 22B is selected, the feature point detection unit 23 detects the feature points from the NIR camera image by using the second detection model 22B. In addition, in a case in which the third detection model 22C is selected, the feature point detection unit 23 detects the feature points from the RGB camera image by using the third detection model 22C. In a case in which the fourth detection model 22D is selected, the feature point detection unit 23 detects the feature points from the NIR camera image by using the fourth detection model 22D.

Then, the imaging range specifying unit 24 determines the detection accuracy of the feature points (step ST45). The imaging range specifying unit 24 specifies the imaging range during the scout imaging based on the feature points in a case in which it is determined that the detection accuracy is high (step ST46), and draws the scan start line and the scan end line during the scout imaging on the schema displayed on the display 14 based on the specified imaging range (line drawing; step ST47). On the other hand, in a case in which it is determined that the detection accuracy is low, the imaging range specifying unit 24 performs the alert display (step ST48).

Then, it is determined whether or not the instruction to start the scout imaging is issued by the operator (step ST49), the processing returns to step ST43 in a case in which a negative determination is made in step ST49, and the processing in and after step ST43 is repeated. In a case in which an affirmative determination is made in step ST49, the camera controller 21 stops the imaging via the camera 7 (step ST50), and the information processing apparatus 10 ends the imaging range specifying processing.

Thereafter, the scout image is acquired by performing the scout imaging via the imaging controller 20, and is displayed on the display 14. The operator checks the scout image, and then sets the imaging range of the main imaging. Then, the operator issues the instruction for the main imaging from the input device 15 to perform the main imaging, and the three-dimensional CT image of the subject H is acquired.

As described above, in the third embodiment, the detection model used for detecting the feature points is selected in accordance with the imaging part in addition to the lightness of the examination room. Therefore, the feature points can be detected by using an appropriate detection model in accordance with the lightness of the examination room and the imaging part. Therefore, the imaging range during the scout imaging can be appropriately specified by using the detected feature points.

It should be noted that, in the third embodiment, both the RGB camera image and the NIR camera image are acquired by the camera 7, but the present invention is not limited thereto. Only the RGB camera image may be acquired by the RGB camera 31 of the camera 7 in a case in which the selection unit 22 makes a determination as being bright, and only the NIR camera image may be acquired by the NIR camera 35 of the camera 7 in a case in which the selection unit 22 makes a determination as being dark.

Next, description regarding a fourth embodiment of the present invention will be made. It should be noted that, since a hardware configuration and a functional configuration of an information processing apparatus according to the fourth embodiment are the same as those of the information processing apparatus according to the third embodiment, the detailed description thereof will be omitted here. The fourth embodiment is different from the third embodiment in that, first, the first detection model 22A that places importance on the frame rate is selected to detect the feature points from the RGB camera image, and then, in accordance with the detection accuracy of the feature points, the feature points are detected from the RGB camera image by using the third detection model 22C that places importance on the accuracy, or the feature points are detected from the NIR camera image by using the fourth detection model 22D that places importance on the accuracy.

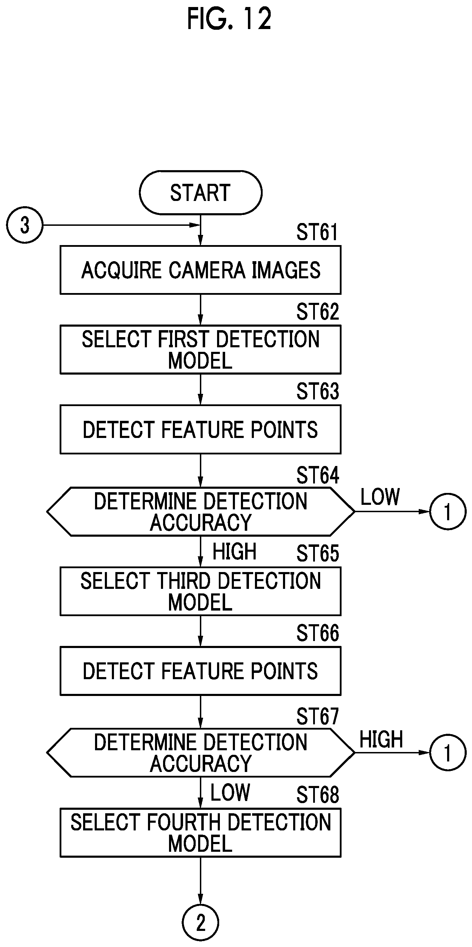

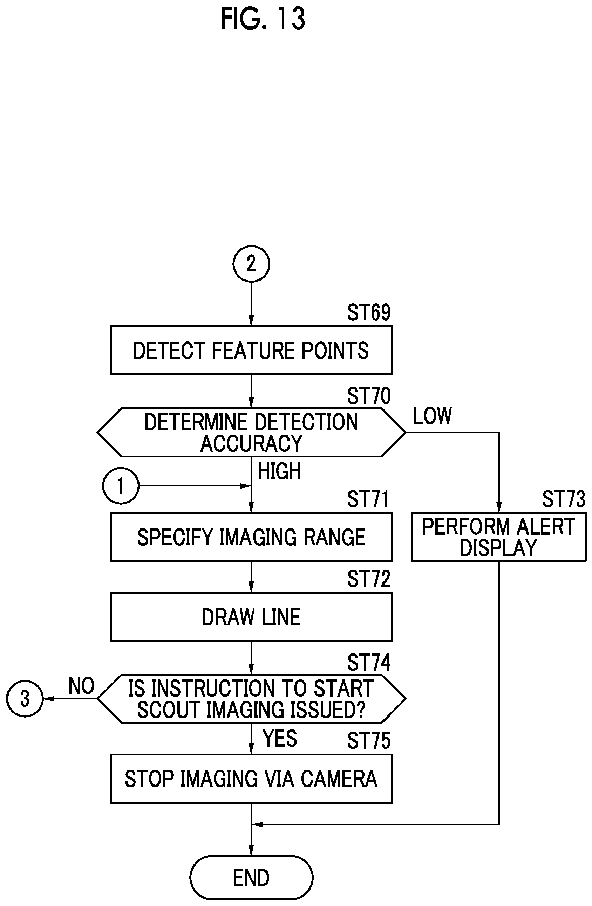

Then, description regarding processing performed in the fourth embodiment will be made. Figs. 12 and 13 are flowcharts showing the processing performed in the third embodiment. For example, the processing is started in response to the instruction to start the imaging from the input device 15, and the camera controller 21 starts the imaging via the camera 7 to acquire the camera images including the RGB camera image and the NIR camera image (step ST61). Next, the selection unit 22 selects the first detection model 22A that places importance on the frame rate (step ST62), and the feature point detection unit 23 detects the feature points from the RGB camera image by using the detection model selected by the selection unit 22 (step ST63).

Next, the imaging range specifying unit 24 determines the detection accuracy of the feature points (step ST64). In a case in which the imaging range specifying unit 24 determines that the detection accuracy is low, the selection unit 22 selects the third detection model 22C that places importance on the accuracy, instead of the first detection model 22A (step ST65). Then, the feature point detection unit 23 detects the feature points from the RGB camera image (step ST66), and the imaging range specifying unit 24 determines the detection accuracy of the feature points (step ST67).

In a case in which the imaging range specifying unit 24 determines that the detection accuracy is low, the selection unit 22 selects the fourth detection model 22D that places importance on the accuracy in a case of detecting the feature points from the NIR camera image, instead of the third detection model 22C (step ST68). It should be noted that, instead of the fourth detection model 22D, the second detection model 22B that places importance on the frame rate in a case of detecting the feature points from the NIR camera image may be selected. Then, the feature point detection unit 23 detects the feature points from the NIR camera image (step ST69), and the imaging range specifying unit 24 determines the detection accuracy of the feature points (step ST70).

The imaging range specifying unit 24 specifies the imaging range during the scout imaging based on the feature points in a case in which it is determined that the detection accuracy is high (step ST71), and draws the scan start line and the scan end line during the scout imaging on the schema displayed on the display 14 based on the specified imaging range (line drawing; step ST72). On the other hand, in a case in which it is determined that the detection accuracy is low, the imaging range specifying unit 24 performs the alert display (step ST73). In a case in which the alert display is performed, the information processing apparatus 10 ends the imaging range specifying processing. In this case, the operator manually sets the imaging range of the scout imaging as described above.

It should be noted that, in a case in which the imaging range specifying unit 24 determines that the detection accuracy is high in step ST64 and step ST67, the processing proceeds to the processing of step ST71, and the processing in and after step ST71 is performed.

Then, it is determined whether or not the instruction to start the scout imaging is issued by the operator (step ST74), the processing returns to step ST61 in a case in which a negative determination is made in step ST74, and the processing in and after step ST61 is repeated. In a case in which an affirmative determination is made in step ST74, the camera controller 21 stops the imaging via the camera 7 (step ST75), and the information processing apparatus 10 ends the imaging range specifying processing.

As described above, in the fourth embodiment, first, the feature points are detected from the RGB camera image by using the first detection model 22A that places importance on the frame rate. In a case in which the detection accuracy of the feature points detected by using the first detection model 22A is high, the imaging range is specified by using the feature points detected by using the first detection model 22A that places importance on the frame rate. In a case in which the detection accuracy of the feature points detected by using the first detection model 22A is low, the feature points are detected from the RGB camera image by using the third detection model 22C that places importance on the accuracy. In a case in which the detection accuracy of the feature points detected by using the third detection model 22C is high, the imaging range is specified by using the feature points detected by using the third detection model 22C. In a case in which the detection accuracy of the feature points detected by using the third detection model 22C is low, the feature points are detected from the NIR camera image by using the fourth detection model 22D that places importance on the accuracy. Therefore, in a case of setting the imaging range, the feature points can be appropriately detected in accordance with the detection accuracy of the feature points, and as a result, the imaging range during the scout imaging can be appropriately specified by using the detected feature points.

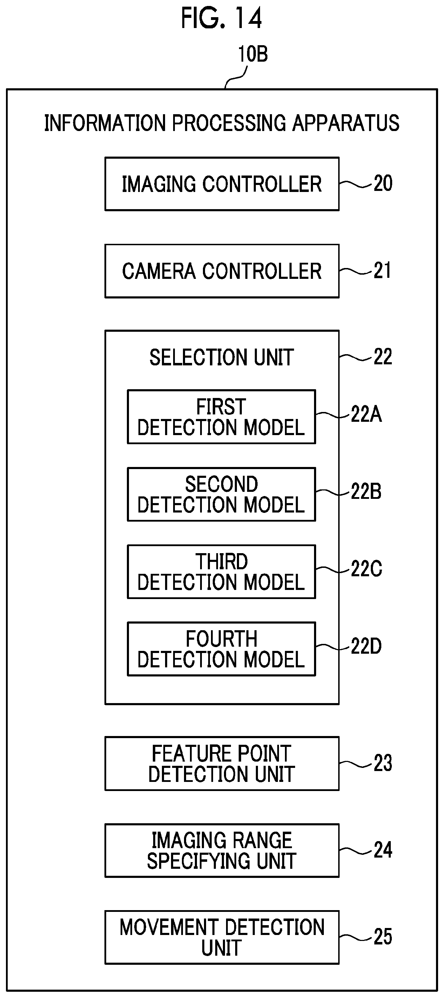

Then, description regarding a fifth embodiment of the present invention will be made. It should be noted that a hardware configuration of an information processing apparatus according to the fifth embodiment is the same as the hardware configuration of the information processing apparatus according to the third embodiment, and thus the detailed description thereof will be omitted here. Fig. 14 is a view showing a functional configuration of the information processing apparatus according to the fifth embodiment. In Fig. 14, the same reference numerals are applied to the same configurations as those in Fig. 10, and the detailed description thereof will be omitted. The fifth embodiment is different from the third embodiment in that an information processing apparatus 10B according to the fifth embodiment comprises a movement detection unit 25 that detects a movement of the subject H.

The movement detection unit 25 detects the movement of the subject H. Specifically, a two-dimensional movement of the feature points detected by using the feature point detection unit 23 is detected between frames that are adjacent in time to each other in the camera image. It should be noted that, in the fifth embodiment, the selection unit 22 first selects the first detection model 22A that places importance on the frame rate, and the feature point detection unit 23 detects the feature points from the RGB camera image by using the first detection model 22A. The feature points for detecting the movement of the subject H may be used in accordance with the imaging part. For example, in a case in which the imaging part is the head, the nose, both eyes, or both ears need only be used, and in a case in which the imaging part is the chest, both shoulders and left and right hip joints need only be used. It should be noted that a representative value of the movements of all the 17 feature points may be obtained as the movement.

The movement detection unit 25 determines that the movement is large in a case in which the detected movement is equal to or larger than a predetermined threshold value, and determines that the movement is small in a case in which the detected movement is smaller than the threshold value. The threshold value for determining the magnitude of the movement is an example of a reference.

In the fifth embodiment, in a case in which the movement detection unit 25 determines that the movement of the subject H is small, the selection unit 22 selects the third detection model 22C that places importance on the accuracy in a case of detecting the feature points from the RGB camera image and the fourth detection model 22D that places importance on the accuracy in a case of detecting the feature points from the NIR camera image, instead of the first detection model 22A. The second detection model 22B that places importance on the frame rate may be selected instead of the fourth detection model 22D. The feature point detection unit 23 detects the feature points by using both the third detection model 22C and the fourth detection model 22D that are selected by the selection unit 22. The imaging range specifying unit 24 specifies the imaging range by using the feature points detected by using the detection model having higher detection accuracy out of the third detection model 22C and the fourth detection model 22D. On the other hand, in a case in which the movement detection unit 25 determines that the movement of the subject H is large, the feature point detection unit 23 continues to use the first detection model 22A used to detect the feature points for detecting the movement to detect the feature points. The imaging range specifying unit 24 specifies the imaging range by using the feature points detected by using the first detection model 22A.

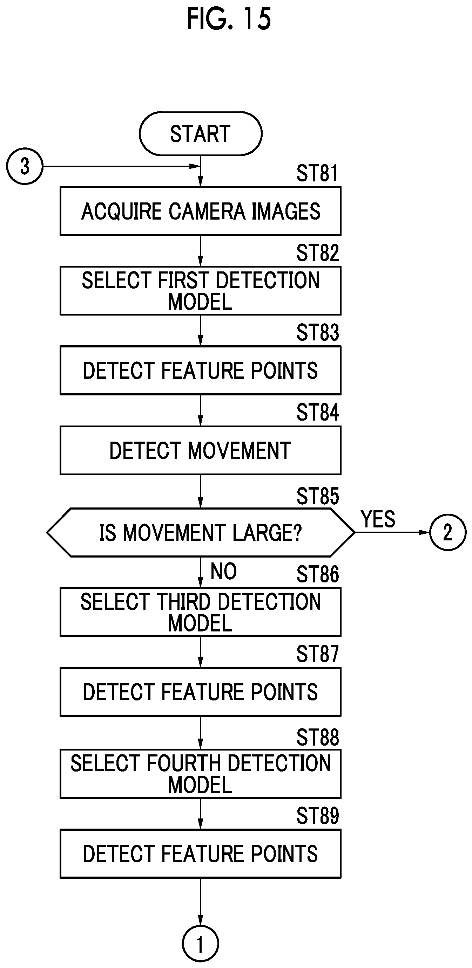

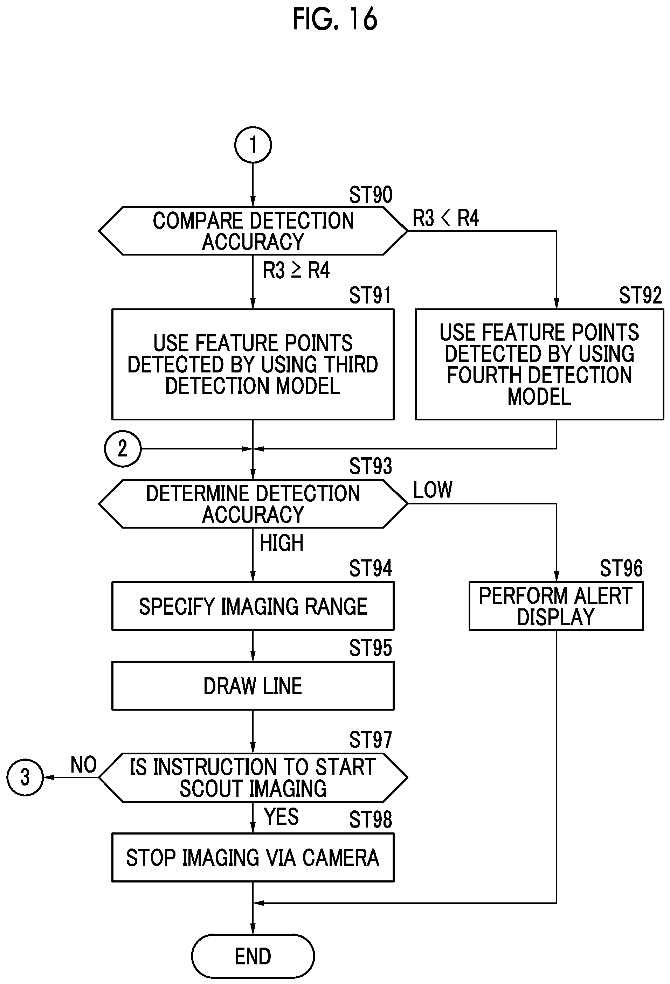

Next, description regarding processing performed in the fifth embodiment will be made. Figs. 15 and 16 are flowcharts showing the processing performed in the fifth embodiment. For example, the processing is started in response to the instruction to start the imaging from the input device 15, and the camera controller 21 starts the imaging via the camera 7 to acquire the camera images including the RGB camera image and the NIR camera image (step ST81). Next, the selection unit 22 selects the first detection model 22A that places importance on the frame rate (step ST82), and the feature point detection unit 23 detects the feature points from the RGB camera image by using the detection model selected by the selection unit 22 (step ST83).

Then, the movement detection unit 25 detects the movement of the subject H by using the feature points detected by using the feature point detection unit 23 (step ST84), and determines whether or not the movement is large (step ST85). In a case in which the movement of the subject H is small and a negative determination is made in step ST85, the selection unit 22 first selects the third detection model 22C that places importance on the accuracy in a case of detecting the feature points from the RGB camera image, instead of the first detection model 22A (step ST86). Then, the feature point detection unit 23 detects the feature points from the RGB camera image (step ST87). Then, the selection unit 22 selects the fourth detection model 22D that places importance on the accuracy in a case of detecting the feature points from the NIR camera image, instead of the third detection model 22C (step ST88). Then, the feature point detection unit 23 detects the feature points from the NIR camera image (step ST89).

It should be noted that the processing of step ST86 and step ST87 and the processing of step ST88 and step ST89 may be performed in parallel, and the processing of step ST88 and step ST89 may be performed before the processing of step ST86 and step ST87.

Next, the imaging range specifying unit 24 compares the detection accuracy of the feature points via the third detection model 22C (referred to as R3) with the detection accuracy of the feature points via the fourth detection model 22D (referred to as R4) (step ST90). Specifically, the imaging range specifying unit 24 compares the representative value of the probabilities for the 17 feature points derived by the third detection model 22C with the representative value of the probabilities for the 17 feature points derived by the fourth detection model 22D. As the representative value, an average value, a median value, a weighted average value corresponding to the imaging part, or the like can be used, but the present invention is not limited thereto.

In a case in which the detection accuracy R3 of the feature points via the third detection model 22C is equal to or higher than the detection accuracy R4 of the feature points via the fourth detection model 22D (R3 $\geq$ R4), the imaging range specifying unit 24 determines to use the feature points detected by using the third detection model 22C for the specification of the imaging range (step ST91). In a case in which the detection accuracy R3 of the feature points via the third detection model 22C is lower than the detection accuracy R4 of the feature points via the fourth detection model 22D (R3 < R4), the imaging range specifying unit 24 determines to use the feature points detected by using the fourth detection model 22D for the specification of the imaging range (step ST92).

Then, the imaging range specifying unit 24 determines the detection accuracy of the feature points (step ST93). It should be noted that, in a case in which an affirmative determination is made in step ST85, the processing proceeds to step ST93, and the imaging range specifying unit 24 determines the detection accuracy of the feature points detected in step ST83.

The imaging range specifying unit 24 specifies the imaging range during the scout imaging based on the feature points in a case in which it is determined that the detection accuracy is high (step ST94), and draws the scan start line and the scan end line during the scout imaging on the schema displayed on the display 14 based on the specified imaging range (line drawing; step ST95). On the other hand, in a case in which it is determined that the detection accuracy is low, the imaging range specifying unit 24 performs the alert display (step ST96). In a case in which the alert display is performed, the information processing apparatus 10 ends the imaging range specifying processing. In this case, the operator manually sets the imaging range of the scout imaging as described above.

Then, it is determined whether or not the instruction to start the scout imaging is issued by the operator (step ST97), the processing returns to step ST81 in a case in which a negative determination is made in step ST97, and the processing in and after step ST81 is repeated. In a case in which an affirmative determination is made in step ST97, the camera controller 21 stops the imaging via the camera 7 (step ST98), and the information processing apparatus 10B ends the imaging range specifying processing.

As described above, in the fifth embodiment, first, the feature points are detected from the RGB camera image by using the first detection model 22A that places importance on the frame rate, and in a case in which the detection accuracy of the feature points detected by using the first detection model 22A is high, the imaging range is specified by using the feature points detected by using the first detection model 22A. In a case in which the detection accuracy of the feature points detected by using the first detection model 22A is low, the feature points are detected from the RGB camera image by using the third detection model 22C that places importance on the accuracy, and the feature points are detected from the NIR camera image by using the fourth detection model 22D that places importance on the accuracy. Then, the imaging range is specified by using the feature points having higher detection accuracy. Therefore, in a case of setting the imaging range, the feature points can be appropriately detected by using the detection model in accordance with the detection accuracy of the feature points, and as a result, the imaging range during the scout imaging can be appropriately specified by using the detected feature points.

It should be noted that, in each of the above-described embodiments, the NIR camera 35 is provided in the camera 7, but the present invention is not limited thereto. A dark vision camera may be used instead of the NIR camera 35. In addition, a camera having higher ISO sensitivity than the RGB camera 31 may be used instead of the NIR camera 35.

In addition, in each of the above-described embodiments, the NIR camera 35 is used as a stereo camera, but the present invention is not limited thereto. Only a single NIR camera may be used.

In addition, in each of the above-described embodiments, the RGB camera 31 is provided in the camera 7, but the present invention is not limited thereto. A camera that can capture a monochrome image may be used instead of the RGB camera 31.

In addition, in the above-described embodiment, the RGB camera 31 and the NIR camera 35 are provided in the camera 7, but the present invention is not limited thereto. The RGB camera 31 and the NIR camera 35 may be separately provided.

In addition, in each of the above-described embodiments, the information processing apparatus according to an embodiment of the present invention is applied to the CT apparatus, but the present invention is not limited thereto. As long as an imaging apparatus acquires the scout image for setting the imaging range before the main imaging, the information processing apparatus according to an embodiment of the present invention may be applied to an MRI apparatus or the like.

In addition, in each of the above-described embodiments, the information processing apparatus comprises the imaging controller 20, but the present invention is not limited thereto. The imaging controller 20 may be provided separately from the information processing apparatus.

In addition, in each of the above-described embodiments, a plurality of detection models having different processing speeds for detecting the feature points may be used for the detection models that place importance on the frame rate. In addition, a plurality of detection models having different accuracy of the feature point detection may be used for the detection models that place importance on the accuracy.

In addition, in the fourth and fifth embodiments, as the detection models that detect the feature points from the NIR camera image, the second detection model 22B that places importance on the frame rate and the fourth detection model 22D that places importance on the accuracy are used, but the present invention is not limited thereto. The information processing apparatus 10B may include only one of the second detection model 22B that places importance on the frame rate or the fourth detection model 22D that places importance on the accuracy. In addition, instead of the second detection model 22B and the fourth detection model 22D, one detection model that can detect the feature points from the NIR camera image at a certain frame rate and certain accuracy may be used.

In addition, in the above-described embodiments, for example, as the hardware structure of the processing units that executes various types of processing, such as the imaging controller 20, the camera controller 21, the selection unit 22, the feature point detection unit 23, the imaging range specifying unit 24, and the movement detection unit 25, various processors described below can be used. As described above, the various processors include, in addition to the CPU that is a general-purpose processor which executes software (program) and functions as various processing units, a programmable logic device (PLD) that is a processor whose circuit configuration can be changed after manufacture, such as a field programmable gate array (FPGA), and a dedicated electrical circuit that is a processor having a circuit configuration which is designed for exclusive use in order to execute specific processing, such as an application specific integrated circuit (ASIC).

One processing unit may be configured by one of these various processors, or may be configured by combining two or more processors of the same type or different types (for example, by combining a plurality of FPGAs or combining of the CPU and the FPGA). A plurality of processing units may be configured by one processor.

As an example of configuring the plurality of processing units by one processor, first, as represented by a computer of a client, a server, and the like there is a form in which one processor is configured by combining one or more CPUs and software and this processor functions as the plurality of processing units. Second, as represented by a system on a chip (SoC) or the like, there is a form of using a processor that realizes the function of the entire system including the plurality of processing units by one integrated circuit (IC) chip. In this way, as the hardware structure, the various processing units are configured by using one or more of the various processors described above.

Further, as the hardware structures of these various processors, more specifically, an electrical circuit (circuitry) in which circuit elements such as semiconductor elements are combined can be used.

**Explanation of References**

1: CT apparatus
2: gantry
3: examination table
3A: examination table part
3B: base part
3C: driving part
4: console
5: opening portion
7: camera
10, 10A, 10B: information processing apparatus
11: CPU
12: information processing program
13: storage
14: display
15: input device
16: memory
17: network I/F
18: bus
20: imaging controller
21: camera controller
22: selection unit
22A: first detection model
22B: second detection model
22C: third detection model
22D: fourth detection model
23: feature point detection unit
24: imaging range specifying unit
25: movement detection unit
31: RGB camera

32: RGB sensor
33: base part
35: NIR camera
36, 37: NIR sensor
38: NIR projector
40: schema
41: start line
42: end line
H: subject

**Claims**

1. An information processing apparatus (10, 10A, 10B) comprising:

   at least one processor (11),
   wherein the processor

   acquires at least one of a first camera image generated by capturing a moving image of a subject on an examination table via a first camera or a second camera image generated by capturing a moving image of the subject via a second camera having higher imaging sensitivity than the first camera,
   selects at least one detection model from among a plurality of detection models including a first detection model (22A) constructed to detect a plurality of feature points on the subject included in the first camera image, and a second detection model (22B) constructed to detect the plurality of feature points on the subject included in the second camera image,
   detects the plurality of feature points on the subject included in the first camera image or in the second camera image by using the selected detection model, and
   specifies an imaging range of the subject based on the plurality of feature points.

2. The information processing apparatus according to claim 1,
   wherein the processor selects the first detection model to detect the plurality of feature points on the subject included in the first camera image in a case in which lightness of an environment in which the examination table is installed is equal to or higher than a reference, and selects the second detection model to detect the plurality of feature points on the subject included in the second camera image in a case in which the lightness is lower than the reference.

3. The information processing apparatus according to claim 2,
   wherein the processor acquires the first camera image and determines the lightness based on brightness information derived from the first camera image.

4. The information processing apparatus according to claim 2 or 3,
wherein the processor acquires the first camera image and determines the lightness based on noise included in the first camera image.

5. The information processing apparatus according to any one of claims 2 to 4,
wherein the processor acquires the first camera image, detects the feature points from the first camera image by using the first detection model, and determines the lightness based on detection accuracy of the feature points.

6. The information processing apparatus according to any one of claims 2 to 5,
wherein the processor determines the lightness by using a sensor that detects the lightness of the environment.

7. The information processing apparatus according to any one of claims 2 to 6,
wherein the processor acquires the first camera image, selects the first detection model to detect the feature points from the first camera image, determines whether or not the lightness is equal to or higher than the reference based on the first camera image, specifies the imaging range based on the feature points detected by using the first detection model in a case in which the lightness is equal to or higher than the reference, selects the second detection model in a case in which the lightness is lower than the reference, and specifies the imaging range based on the feature points detected by using the second detection model.

8. The information processing apparatus according to claim 1,

wherein the first detection model and the second detection model are models that place importance on a frame rate in a case of detecting the feature points,
the plurality of detection models further include a third detection model that is constructed to detect the plurality of feature points on the subject included in the first camera image and that places importance on accuracy in a case of detecting the feature points, and a fourth detection model that is constructed to detect the plurality of feature points on the subject included in the second camera image and that places importance on accuracy in a case of detecting the feature points, and

the processor selects the detection model in accordance with lightness of an environment in which the examination table is installed and an imaging part of the subject.

9. The information processing apparatus according to claim 8,
wherein the processor selects any one of the first detection model or the third detection model to detect the plurality of feature points on the subject included in the first camera image in a case in which the lightness is equal to or higher than a reference, and selects any one of the second detection model or the fourth detection model to detect the plurality of feature points on the subject included in the second camera image in a case in which the lightness is lower than the reference.

10. The information processing apparatus according to claim 8 or 9,
wherein the processor selects any one of the first detection model or the third detection model and any one of the second detection model or the fourth detection model, in accordance with the imaging part of the subject.

11. The information processing apparatus according to any one of claims 1 to 10,
wherein the processor determines detection accuracy of the feature points, specifies the imaging range based on the feature points in a case in which the detection accuracy is equal to or higher than a reference, and issues an alert in a case in which the detection accuracy is lower than the reference.

12. The information processing apparatus according to claim 1,

wherein the first detection model is a model that places importance on a frame rate in a case of detecting the feature points,
the plurality of detection models further include a third detection model that is constructed to detect the plurality of feature points on the subject included in the first camera image and that places importance on accuracy in a case of detecting the feature points, and
the processor

acquires the first camera image and the second camera image and selects the first detection model to detect the feature points from the first camera image,
determines detection accuracy of the feature points detected by using the first detection model, specifies the imaging range based on the feature points detected by using the first detection model in a case in which the detection accuracy is equal to or higher than a first reference,

selects the third detection model to detect the feature points from the first camera image by using the third detection model in a case in which the detection accuracy is lower than the first reference,

determines detection accuracy of the feature points detected by using the third detection model,

specifies the imaging range based on the feature points detected by using the third detection model in a case in which the detection accuracy is equal to or higher than a second reference,

selects the second detection model to detect the feature points from the second camera image by using the second detection model in a case in which the detection accuracy is lower than the second reference, and

specifies the imaging range based on the feature points detected by using the second detection model.

13. The information processing apparatus according to claim 12,
    wherein the processor

determines the detection accuracy of the feature points detected by using the first detection model in a case in which the detection accuracy is equal to or higher than the first reference, the detection accuracy of the feature points detected by using the third detection model in a case in which the detection accuracy is equal to or higher than the second reference, or detection accuracy of the feature points detected by using the second detection model,

specifies, in a case in which the detection accuracy is equal to or higher than a third reference, the imaging range based on the feature points detected by using the first detection model in a case in which the detection accuracy is equal to or higher than the first reference, the feature points detected by using the third detection model in a case in which the detection accuracy is equal to or higher than the second reference, or the feature points detected by using the second detection model, and

issues an alert in a case in which the detection accuracy is lower than the third reference.

14. The information processing apparatus according to claim 1,

wherein the first detection model is a model that places importance on a frame rate in a case of detecting the feature points,

the plurality of detection models further include a third detection model that is constructed to detect the plurality of feature points on the subject included in the first camera image and that places importance on accuracy in a case of detecting the feature points, and
the processor

acquires the first camera image and the second camera image and selects the first detection model to detect the feature points from the first camera image,

detects a movement of the subject based on the first camera image,

specifies the imaging range based on the feature points detected by using the first detection model in a case in which the movement of the subject is equal to or larger than a first reference,

selects the second detection model and the third detection model in a case in which the movement of the subject is smaller than the first reference,

detects the feature points from the first camera image by using the third detection model,

detects the feature points from the second camera image by using the second detection model,

compares detection accuracy of the feature points detected by using the third detection model with detection accuracy of the feature points detected by using the second detection model,

specifies the imaging range based on the feature points detected by using the third detection model in a case in which the detection accuracy of the feature points detected by using the third detection model is higher, and

specifies the imaging range based on the feature points detected by using the second detection model in a case in which the detection accuracy of the feature points detected by using the second detection model is higher.

15. The information processing apparatus according to claim 14,
    wherein the processor

determines detection accuracy of the feature points detected by using the first detection model in a case in which the movement is equal to or larger than the first reference, the detection accuracy of the feature points detected by using the third detection model, or the detection accuracy of the feature points detected by using the second detection model,

specifies, in a case in which the detection accuracy is equal to or higher than a second reference, the imaging range based on the feature points detected by using the first detection model in a case in which the movement is equal to or larger than the first reference, the feature points detected by using the third detection model, or the feature points detected by using the second detection model, and

issues an alert in a case in which the detection accuracy is lower than the second reference.

16. The information processing apparatus according to any one of claims 1 to 15,

wherein the processor derives a movement range of the examination table based on the imaging range.

17. The information processing apparatus according to claim 16,

wherein the processor displays, on a display, a human body image imitating a human body and draws, on the human body image, a movement start line and a movement end line of the examination table based on the movement range of the examination table.

18. The information processing apparatus according to any one of claims 1 to 17,

wherein the imaging range is an imaging range in a case of capturing an image for positioning acquired before main imaging of the subject is performed.

19. An information processing method comprising:

via a computer,

acquiring at least one of a first camera image generated by capturing a moving image of a subject on an examination table via a first camera or a second camera image generated by capturing a moving image of the subject via a second camera having higher imaging sensitivity than the first camera;

selecting at least one detection model from among a plurality of detection models including a first detection model constructed to detect a plurality of feature points on the subject included in the first camera image, and a second detection model constructed to detect the plurality of feature points on the subject included in the second camera image;

detecting the plurality of feature points on the subject included in the first camera image or in the second camera image by using the selected detection model; and

specifying an imaging range of the subject based on the plurality of feature points.

20. A computer-readable storage medium that stores an information processing program causing a computer to execute:

a procedure of acquiring at least one of a first camera image generated by capturing a moving image of a subject on an examination table via a first camera or a second camera image generated by capturing a moving image of the subject via a second camera having higher imaging sensitivity than the first camera;

a procedure of selecting at least one detection model from among a plurality of detection models including a first detection model constructed to detect a plurality of feature points on the subject included in the first camera image, and a second detection model constructed to detect the plurality of feature points on the subject included in the second camera image;

a procedure of detecting the plurality of feature points on the subject included in the first camera image or in the second camera image by using the selected detection model; and

a procedure of specifying an imaging range of the subject based on the plurality of feature points.

**Patentansprüche**

1. Informationsverarbeitungsvorrichtung (10, 10A, 10B), umfassend:

mindestens einen Prozessor (11), wobei der Prozessor

mindestens eines von einem ersten Kamerabild, das durch Aufnehmen eines Bewegtbildes einer Untersuchungsperson auf einem Untersuchungstisch via eine erste Kamera erzeugt wird, und einem zweiten Kamerabild, das durch Aufnehmen eines Bewegtbildes der Untersuchungsperson via eine zweite Kamera, die höhere Bildgebungsempfindlichkeit als die erste Kamera aufweist, erzeugt wird, erfasst,

mindestens ein Detektionsmodell aus mehreren Detektionsmodellen, die ein erstes Detektionsmodell (22A), das so konstruiert ist, dass es mehrere Merkmalspunkte auf der Untersuchungsperson, die in dem ersten Kamerabild enthalten sind, detektiert, und ein zweites Detektionsmodell (22B), das so konstruiert ist, dass es die mehreren Merkmalspunkte auf der Untersuchungsperson, die in dem zweiten Kamerabild enthalten sind, detektiert, enthalten, auswählt,

die mehreren Merkmalspunkte auf der Untersuchungsperson, die in dem ersten Kamerabild oder in dem zweiten Kamerabild enthalten sind, durch Verwenden des ausgewählten Detektionsmodells detektiert, und
einen Bildgebungsbereich der Untersuchungsperson auf der Grundlage der mehreren Merkmalspunkte spezifiziert.

2. Informationsverarbeitungsvorrichtung nach Anspruch 1,
wobei der Prozessor das erste Detektionsmodell auswählt, um die mehreren Merkmalspunkte auf der Untersuchungsperson, die in dem ersten Kamerabild enthalten sind, in einem Fall zu detektieren, in dem Helligkeit einer Umgebung, in der der Untersuchungstisch installiert ist, gleich oder höher als eine Referenz ist, und das zweite Detektionsmodell auswählt, um die mehreren Merkmalspunkte auf der Untersuchungsperson, die in dem zweiten Kamerabild enthalten sind, in einem Fall zu detektieren, in dem die Helligkeit niedriger als die Referenz ist.

3. Informationsverarbeitungsvorrichtung nach Anspruch 2,
wobei der Prozessor das erste Kamerabild erfasst und die Helligkeit auf der Grundlage von von dem ersten Kamerabild abgeleiteten Helligkeitsinformationen bestimmt.

4. Informationsverarbeitungsvorrichtung nach Anspruch 2 oder 3,
wobei der Prozessor das erste Kamerabild erfasst und die Helligkeit auf der Grundlage von Rauschen, das in dem ersten Kamerabild enthalten ist, bestimmt.

5. Informationsverarbeitungsvorrichtung nach einem der Ansprüche 2 bis 4,
wobei der Prozessor das erste Kamerabild erfasst, die Merkmalspunkte aus dem ersten Kamerabild durch Verwenden des ersten Detektionsmodells detektiert, und die Helligkeit auf der Grundlage von Detektionsgenauigkeit der Merkmalspunkte bestimmt.

6. Informationsverarbeitungsvorrichtung nach einem der Ansprüche 2 bis 5,
wobei der Prozessor die Helligkeit durch Verwenden eines Sensors, der die Helligkeit der Umgebung detektiert, bestimmt.

7. Informationsverarbeitungsvorrichtung nach einem der Ansprüche 2 bis 6,
wobei der Prozessor das erste Kamerabild erfasst, das erste Detektionsmodell auswählt, um die Merkmalspunkte aus dem ersten Kamerabild zu detektieren, auf der Grundlage des ersten Kamerabildes bestimmt, ob die Helligkeit gleich oder höher als die Referenz ist oder nicht, den Bildgebungsbereich auf der Grundlage der Merkmalspunkte, die durch Verwenden des ersten Detektionsmodells detektiert werden, in einem Fall, in dem die Helligkeit gleich oder höher als die Referenz ist, spezifiziert, das zweite Detektionsmodell in einem Fall, in dem die Helligkeit niedriger als die Referenz ist, auswählt, und den Bildgebungsbereich auf der Grundlage der Merkmalspunkte, die durch Verwenden des zweiten Detektionsmodells detektiert werden, spezifiziert.

8. Informationsverarbeitungsvorrichtung nach Anspruch 1,

wobei das erste Detektionsmodell und das zweite Detektionsmodell Modelle sind, die in einem Fall des Detektierens der Merkmalspunkte auf eine Bildrate Wert legen,
die mehreren Detektionsmodelle ferner ein drittes Detektionsmodell, das so konstruiert ist, dass es die mehreren Merkmalspunkte auf der Untersuchungsperson, die in dem ersten Kamerabild enthalten sind, detektiert und das in einem Fall des Detektierens der Merkmalspunkte auf Genauigkeit Wert legt, und ein viertes Detektionsmodell, das so konstruiert ist, dass es die mehreren Merkmalspunkte auf der Untersuchungsperson, die in dem zweiten Kamerabild enthalten sind, detektiert und das in einem Fall des Detektierens der Merkmalspunkte auf Genauigkeit Wert legt, enthalten, und
der Prozessor das Detektionsmodell in Übereinstimmung mit Helligkeit einer Umgebung, in der der Untersuchungstisch installiert ist, und einem Bildgebungsteil der Untersuchungsperson auswählt.

9. Informationsverarbeitungsvorrichtung nach Anspruch 8,
wobei der Prozessor das erste Detektionsmodell oder das dritte Detektionsmodell auswählt, um die mehreren Merkmalspunkte auf der Untersuchungsperson, die in dem ersten Kamerabild enthalten sind, in einem Fall zu detektieren, in dem die Helligkeit gleich oder höher als eine Referenz ist, und das zweite Detektionsmodell oder das vierte Detektionsmodell auswählt, um die mehreren Merkmalspunkte auf der Untersuchungsperson, die in dem zweiten Kamerabild enthalten sind, in einem Fall zu detektieren, in dem die Helligkeit niedriger als die Referenz ist.

10. Informationsverarbeitungsvorrichtung nach Anspruch 8 oder 9,
wobei der Prozessor das erste Detektionsmodell oder das dritte Detektionsmodell und das zweite Detektionsmodell oder das vierte Detektionsmodell in Übereinstimmung mit dem Bildgebungsteil der Untersuchungsperson auswählt.

**11.** Informationsverarbeitungsvorrichtung nach einem der Ansprüche 1 bis 10,
wobei der Prozessor Detektionsgenauigkeit der Merkmalspunkte bestimmt, den Bildgebungsbereich auf der Grundlage der Merkmalspunkte in einem Fall, in dem die Detektionsgenauigkeit gleich oder höher als eine Referenz ist, spezifiziert, und in einem Fall, in dem die Detektionsgenauigkeit niedriger als die Referenz ist, eine Warnung ausgibt.

**12.** Informationsverarbeitungsvorrichtung nach Anspruch 1,

wobei das erste Detektionsmodell ein Modell ist, das in einem Fall des Detektierens der Merkmalspunkte auf eine Bildrate Wert legt,
die mehreren Detektionsmodelle ferner ein drittes Detektionsmodell, das so konstruiert ist, dass es die mehreren Merkmalspunkte auf der Untersuchungsperson, die in dem ersten Kamerabild enthalten sind, detektiert und das in einem Fall des Detektierens der Merkmalspunkte auf Genauigkeit Wert legt, enthalten, und der Prozessor
das erste Kamerabild und das zweite Kamerabild erfasst und das erste Detektionsmodell auswählt, um die Merkmalspunkte aus dem ersten Kamerabild zu detektieren,
Detektionsgenauigkeit der Merkmalspunkte, die durch Verwenden des ersten Detektionsmodells detektiert werden, bestimmt,
den Bildgebungsbereich auf der Grundlage der Merkmalspunkte, die durch Verwenden des ersten Detektionsmodells detektiert werden, in einem Fall, in dem die Detektionsgenauigkeit gleich oder höher als eine erste Referenz ist, spezifiziert,
das dritte Detektionsmodell auswählt, um die Merkmalspunkte aus dem ersten Kamerabild durch Verwenden des dritten Detektionsmodells in einem Fall, in dem die Detektionsgenauigkeit niedriger als die erste Referenz ist, zu detektieren,
Detektionsgenauigkeit der Merkmalspunkte, die durch Verwenden des dritten Detektionsmodells detektiert werden, bestimmt,
den Bildgebungsbereich auf der Grundlage der Merkmalspunkte, die durch Verwenden des dritten Detektionsmodells detektiert werden, in einem Fall, in dem die Detektionsgenauigkeit gleich oder höher als eine zweite Referenz ist, spezifiziert,
das zweite Detektionsmodell auswählt, um die Merkmalspunkte aus dem zweiten Kamerabild durch Verwenden des zweiten Detektionsmodells in einem Fall, in dem die Detektionsgenauigkeit niedriger als die zweite Referenz ist, zu detektieren, und den Bildgebungsbereich auf der Grundlage der Merkmalspunkte, die durch Verwenden des zweiten Detektionsmodells detektiert werden, spezifiziert.

**13.** Informationsverarbeitungsvorrichtung nach Anspruch 12,

wobei der Prozessor
in einem Fall, in dem die Detektionsgenauigkeit gleich oder höher als eine erste Referenz ist, die Detektionsgenauigkeit der Merkmalspunkte, die durch Verwenden des ersten Detektionsmodells detektiert werden, in einem Fall, in dem die Detektionsgenauigkeit gleich oder höher als eine zweite Referenz ist, die Detektionsgenauigkeit der Merkmalspunkte, die durch Verwenden des dritten Detektionsmodells detektiert werden, oder Detektionsgenauigkeit der Merkmalspunkte, die durch Verwenden des zweiten Detektionsmodells detektiert werden, bestimmt,
in einem Fall, in dem die Detektionsgenauigkeit gleich oder höher als eine dritte Referenz ist, den Bildgebungsbereich auf der Grundlage der Merkmalspunkte, die durch Verwenden des ersten Detektionsmodells detektiert werden, in einem Fall, in dem die Detektionsgenauigkeit gleich oder höher als die erste Referenz ist, der Merkmalspunkte, die durch Verwenden des dritten Detektionsmodells detektiert werden, in einem Fall, in dem die Detektionsgenauigkeit gleich oder höher als die zweite Referenz ist, oder der Merkmalspunkte, die durch Verwenden des zweiten Detektionsmodells detektiert werden, spezifiziert, und
in einem Fall, in dem die Detektionsgenauigkeit niedriger als die dritte Referenz ist, eine Warnung ausgibt.

**14.** Informationsverarbeitungsvorrichtung nach Anspruch 1,

wobei das erste Detektionsmodell ein Modell ist, das in einem Fall des Detektierens der Merkmalspunkte auf eine Bildrate Wert legt,
die mehreren Detektionsmodelle ferner ein drittes Detektionsmodell, das so konstruiert ist, dass es die mehreren Merkmalspunkte auf der Untersuchungsperson, die in dem ersten Kamerabild enthalten sind, detektiert und das in einem Fall des Detektierens der Merkmalspunkte auf Genauigkeit Wert legt, enthalten, und der Prozessor
das erste Kamerabild und das zweite Kamerabild erfasst und das erste Detektionsmodell auswählt, um die Merkmalspunkte aus dem ersten Kamerabild zu detektieren,
eine Bewegung der Untersuchungsperson auf der Grundlage des ersten Kamerabilds detektiert,

den Bildgebungsbereich auf der Grundlage der Merkmalspunkte, die durch Verwenden des ersten Detektionsmodells detektiert werden, in einem Fall, in dem die Bewegung der Untersuchungsperson gleich oder größer als eine erste Referenz ist, spezifiziert,

das zweite Detektionsmodell und das dritte Detektionsmodell in einem Fall, in dem die Bewegung der Untersuchungsperson kleiner als die erste Referenz ist, auswählt, die Merkmalspunkte aus dem ersten Kamerabild durch Verwenden des dritten Detektionsmodells detektiert,

die Merkmalspunkte aus dem zweiten Kamerabild durch Verwenden des zweiten Detektionsmodells detektiert,

Detektionsgenauigkeit der Merkmalspunkte, die durch Verwenden des dritten Detektionsmodells detektiert werden, mit Detektionsgenauigkeit der Merkmalspunkte, die durch Verwenden des zweiten Detektionsmodells detektiert werden, vergleicht, den Bildgebungsbereich auf der Grundlage der Merkmalspunkte, die durch Verwenden des dritten Detektionsmodells detektiert werden, in einem Fall, in dem die Detektionsgenauigkeit der Merkmalspunkte, die durch Verwenden des dritten Detektionsmodells detektiert werden, höher ist, spezifiziert, und

den Bildgebungsbereich auf der Grundlage der Merkmalspunkte, die durch Verwenden des zweiten Detektionsmodells detektiert werden, in einem Fall, in dem die Detektionsgenauigkeit der Merkmalspunkte, die durch Verwenden des zweiten Detektionsmodells detektiert werden, höher ist, spezifiziert.

15. Informationsverarbeitungsvorrichtung nach Anspruch 14,

wobei der Prozessor
in einem Fall, in dem die Bewegung gleich oder größer als die erste Referenz ist, die Detektionsgenauigkeit der Merkmalspunkte, die durch Verwenden des ersten Detektionsmodells detektiert werden, die Detektionsgenauigkeit der Merkmalspunkte, die durch Verwenden des dritten Detektionsmodells detektiert werden, oder die Detektionsgenauigkeit der Merkmalspunkte, die durch Verwenden des zweiten Detektionsmodells detektiert werden, bestimmt,
in einem Fall, in dem die Detektionsgenauigkeit gleich oder höher als eine zweite Referenz ist, den Bildgebungsbereich auf der Grundlage der Merkmalspunkte, die durch Verwenden des ersten Detektionsmodells detektiert werden, in einem Fall, in dem die Bewegung gleich oder größer als die erste Referenz ist, der Merkmalspunkte, die durch Verwenden des dritten Detektionsmodells detektiert werden, oder der Merkmalspunkte, die durch Verwenden des zweiten Detektionsmodells detektiert werden, spezifiziert, und
in einem Fall, in dem die Detektionsgenauigkeit niedriger als die zweite Referenz ist, eine Warnung ausgibt.

16. Informationsverarbeitungsvorrichtung nach einem der Ansprüche 1 bis 15,
wobei der Prozessor einen Bewegungsbereich des Untersuchungstisches auf der Grundlage des Bildgebungsbereichs ableitet.

17. Informationsverarbeitungsvorrichtung nach Anspruch 16,
wobei der Prozessor ein menschliches Körperbild, das einen menschlichen Körper imitiert, auf einer Anzeige anzeigt und auf dem menschlichen Körperbild eine Bewegungsstartlinie und eine Bewegungsendlinie des Untersuchungstisches auf der Grundlage des Bewegungsbereichs des Untersuchungstisches zeichnet.

18. Informationsverarbeitungsvorrichtung nach einem der Ansprüche 1 bis 17,
wobei der Bildgebungsbereich ein Bildgebungsbereich in einem Fall des Aufnehmens eines Bildes zum Positionieren ist, das erfasst wird, bevor Hauptbildgebung der Untersuchungsperson durchgeführt wird.

19. Informationsverarbeitungsverfahren, umfassend:

via einen Computer,
Erfassen von mindestens einem von einem ersten Kamerabild, das durch Aufnehmen eines Bewegtbildes einer Untersuchungsperson auf einem Untersuchungstisch via eine erste Kamera erzeugt wird, und einem zweiten Kamerabild, das durch Aufnehmen eines Bewegtbildes der Untersuchungsperson via eine zweite Kamera, die höhere Bildgebungsempfindlichkeit als die erste Kamera aufweist, erzeugt wird;
Auswählen von mindestens einem Detektionsmodell aus mehreren Detektionsmodellen, die ein erstes Detektionsmodell, das so konstruiert ist, dass es mehrere Merkmalspunkte auf der Untersuchungsperson, die in dem ersten Kamerabild enthalten sind, detektiert, und ein zweites Detektionsmodell, das so konstruiert ist, dass es die mehreren Merkmalspunkte auf der Untersuchungsperson, die in dem zweiten Kamerabild enthalten sind, detektiert, enthalten;
Detektieren der mehreren Merkmalspunkte auf der Untersuchungsperson, die in dem ersten Kamerabild oder in dem zweiten Kamerabild enthalten sind, durch Verwenden des ausgewählten Detektionsmodells; und

Spezifizieren eines Bildgebungsbereichs der Untersuchungsperson auf der Grundlage der mehreren Merkmalspunkte.

20. Computerlesbares Speichermedium, das ein Informationsverarbeitungsprogramm speichert, das einen Computer veranlasst, auszuführen:

ein Verfahren des Erfassens von mindestens einem von einem ersten Kamerabild, das durch Aufnehmen eines Bewegtbildes einer Untersuchungsperson auf einem Untersuchungstisch via eine erste Kamera erzeugt wird, und einem zweiten Kamerabild, das durch Aufnehmen eines Bewegtbildes der Untersuchungsperson via eine zweite Kamera, die höhere Bildgebungsempfindlichkeit als die erste Kamera aufweist, erzeugt wird;

ein Verfahren des Auswählens von mindestens einem Detektionsmodell aus mehreren Detektionsmodellen, die ein erstes Detektionsmodell, das so konstruiert ist, dass es mehrere Merkmalspunkte auf der Untersuchungsperson, die in dem ersten Kamerabild enthalten sind, detektiert, und ein zweites Detektionsmodell, das so konstruiert ist, dass es die mehreren Merkmalspunkte auf der Untersuchungsperson, die in dem zweiten Kamerabild enthalten sind, detektiert, enthalten;

ein Verfahren des Detektierens der mehreren Merkmalspunkte auf der Untersuchungsperson, die in dem ersten Kamerabild oder in dem zweiten Kamerabild enthalten sind, durch Verwenden des ausgewählten Detektionsmodells; und

ein Verfahren des Spezifizierens eines Bildgebungsbereichs der Untersuchungsperson auf der Grundlage der mehreren Merkmalspunkte.

**Revendications**

1. Appareil de traitement d'informations (10, 10A, 10B) comprenant :

au moins un processeur (11),
dans lequel le processeur
acquiert au moins l'une d'une première image de caméra générée en capturant une image en mouvement d'un sujet sur une table d'examen via une première caméra ou
d'une deuxième image de caméra générée en capturant une image en mouvement du sujet via une deuxième caméra ayant une sensibilité d'imagerie supérieure à celle de la première caméra,
sélectionne au moins un modèle de détection parmi une pluralité de modèles de détection incluant un premier modèle de détection (22A) construit pour détecter une pluralité de points caractéristiques sur le sujet inclus dans la première image de caméra, et un deuxième modèle de détection (22B) construit pour détecter la pluralité de points caractéristiques sur le sujet inclus dans la deuxième image de caméra, détecte la pluralité de points caractéristiques sur le sujet inclus dans la première image de caméra ou dans la deuxième image de caméra en utilisant le modèle de détection sélectionné, et spécifie une plage d'imagerie du sujet sur la base de la pluralité de points caractéristiques.

2. Appareil de traitement d'informations selon la revendication 1,
dans lequel le processeur sélectionne le premier modèle de détection pour détecter la pluralité de points caractéristiques sur le sujet inclus dans la première image de caméra dans un cas où la luminosité d'un environnement dans lequel la table d'examen est installée est égale ou supérieure à une référence, et sélectionne le deuxième modèle de détection pour détecter la pluralité de points caractéristiques sur le sujet inclus dans la deuxième image de caméra dans un cas où la luminosité est inférieure à la référence.

3. Appareil de traitement d'informations selon la revendication 2,
dans lequel le processeur acquiert la première image de caméra et détermine la luminosité sur la base d'informations de luminosité dérivées de la première image de caméra.

4. Appareil de traitement d'informations selon la revendication 2 ou la revendication 3, dans lequel le processeur acquiert la première image de caméra et détermine la luminosité sur la base d'un bruit inclus dans la première image de caméra.

5. Appareil de traitement d'informations selon l'une quelconque des revendications 2 à 4, dans lequel le processeur acquiert la première image de caméra, détecte les points caractéristiques à partir de la première image de caméra en utilisant le premier modèle de détection, et détermine la luminosité sur la base d'une précision de détection des points caractéristiques.

6. Appareil de traitement d'informations selon l'une quelconque des revendications 2 à 5, dans lequel le processeur détermine la luminosité en utilisant un capteur qui détecte la luminosité de l'environnement.

**7.** Appareil de traitement d'informations selon l'une quelconque des revendications 2 à 6, dans lequel le processeur acquiert la première image de caméra, sélectionne le premier modèle de détection pour détecter les points caractéristiques à partir de la première image de caméra, détermine si oui ou non la luminosité est égale ou supérieure à la référence sur la base de la première image de caméra, spécifie la plage d'imagerie sur la base des points caractéristiques détectés en utilisant le premier modèle de détection dans un cas où la luminosité est égale ou supérieure à la référence, sélectionne le deuxième modèle de détection dans un cas où la luminosité est inférieure à la référence, et spécifie la plage d'imagerie sur la base des points caractéristiques détectés en utilisant le deuxième modèle de détection.

**8.** Appareil de traitement d'informations selon la revendication 1,

dans lequel le premier modèle de détection et le deuxième modèle de détection sont des modèles qui accordent une importance à une fréquence d'images dans un cas de détection des points caractéristiques,
la pluralité de modèles de détection inclut en outre un troisième modèle de détection qui est construit pour détecter la pluralité de points caractéristiques sur le sujet inclus dans la première image de caméra et qui accorde une importance à une précision dans un cas de détection des points caractéristiques, et un quatrième modèle de détection qui est construit pour détecter la pluralité de points caractéristiques sur le sujet inclus dans la deuxième image de caméra et qui accorde une importance à une précision dans un cas de détection des points caractéristiques, et
le processeur sélectionne le modèle de détection conformément à la luminosité d'un environnement dans lequel la table d'examen est installée et à une partie d'imagerie du sujet.

**9.** Appareil de traitement d'informations selon la revendication 8,
dans lequel le processeur sélectionne l'un quelconque du premier modèle de détection ou du troisième modèle de détection pour détecter la pluralité de points caractéristiques sur le sujet inclus dans la première image de caméra dans un cas où la luminosité est égale ou supérieure à une référence, et sélectionne l'un quelconque du deuxième modèle de détection ou du quatrième modèle de détection pour détecter la pluralité de points caractéristiques sur le sujet inclus dans la deuxième image de caméra dans un cas où la luminosité est inférieure à la référence.

**10.** Appareil de traitement d'informations selon la revendication 8 ou la revendication 9, dans lequel le processeur sélectionne l'un quelconque du premier modèle de détection ou du troisième modèle de détection et l'un quelconque du deuxième modèle de détection ou du quatrième modèle de détection, conformément à la partie d'imagerie du sujet.

**11.** Appareil de traitement d'informations selon l'une quelconque des revendications 1 à 10,
dans lequel le processeur détermine une précision de détection des points caractéristiques, spécifie la plage d'imagerie sur la base des points caractéristiques dans un cas où la précision de détection est égale ou supérieure à une référence, et émet une alerte dans un cas où la précision de détection est inférieure à la référence.

**12.** Appareil de traitement d'informations selon la revendication 1,

dans lequel le premier modèle de détection est un modèle qui accorde une importance à une fréquence d'images dans un cas de détection des points caractéristiques,
la pluralité de modèles de détection inclut en outre un troisième modèle de détection qui est construit pour détecter la pluralité de points caractéristiques sur le sujet inclus dans la première image de caméra et qui accorde une importance à une précision dans un cas de détection des points caractéristiques, et
le processeur
acquiert la première image de caméra et la deuxième image de caméra et sélectionne le premier modèle de détection pour détecter les points caractéristiques à partir de la première image de caméra,
détermine une précision de détection des points caractéristiques détectés en utilisant le premier modèle de détection,
spécifie la plage d'imagerie sur la base des points caractéristiques détectés en utilisant le premier modèle de détection dans un cas où la précision de détection est égale ou supérieure à une première référence,
sélectionne le troisième modèle de détection pour détecter les points caractéristiques à partir de la première image de caméra en utilisant le troisième modèle de détection dans un cas où la précision de détection est inférieure à la première référence,
détermine une précision de détection des points caractéristiques détectés en utilisant le troisième modèle de détection,
spécifie la plage d'imagerie sur la base des points caractéristiques détectés en utilisant le troisième modèle de détection dans un cas où la précision de détection est égale ou supérieure à une deuxième référence,

sélectionne le deuxième modèle de détection pour détecter les points caractéristiques à partir de la deuxième image de caméra en utilisant le deuxième modèle de détection dans un cas où la précision de détection est inférieure à la deuxième référence, et spécifie la plage d'imagerie sur la base des points caractéristiques détectés en utilisant le deuxième modèle de détection.

13. Appareil de traitement d'informations selon la revendication 12,

dans lequel le processeur
détermine la précision de détection des points caractéristiques détectés en utilisant le premier modèle de détection dans un cas où la précision de détection est égale ou supérieure à la première référence, la précision de détection des points caractéristiques détectés en utilisant le troisième modèle de détection dans un cas où la précision de détection est égale ou supérieure à la deuxième référence, ou une précision de détection des points caractéristiques détectés en utilisant le deuxième modèle de détection,
spécifie, dans un cas où la précision de détection est égale ou supérieure à une troisième référence, la plage d'imagerie sur la base des points caractéristiques détectés en utilisant le premier modèle de détection dans un cas où la précision de détection est égale ou supérieure à la première référence, des points caractéristiques détectés en utilisant le troisième modèle de détection dans un cas où la précision de détection est égale ou supérieure à la deuxième référence, ou des points caractéristiques détectés en utilisant le deuxième modèle de détection, et
émet une alerte dans un cas où la précision de détection est inférieure à la troisième référence.

14. Appareil de traitement d'informations selon la revendication 1,

dans lequel le premier modèle de détection est un modèle qui accorde une importance à une fréquence d'images dans un cas de détection des points caractéristiques,
la pluralité de modèles de détection inclut en outre un troisième modèle de détection qui est construit pour détecter la pluralité de points caractéristiques sur le sujet inclus dans la première image de caméra et qui accorde une importance à une précision dans un cas de détection des points caractéristiques, et
le processeur
acquiert la première image de caméra et la deuxième image de caméra et sélectionne le premier modèle de détection pour détecter les points caractéristiques à partir de la première image de caméra,
détecte un mouvement du sujet sur la base de la première image de caméra,
spécifie la plage d'imagerie sur la base des points caractéristiques détectés en utilisant le premier modèle de détection dans un cas où le mouvement du sujet est égal ou supérieur à une première référence,
sélectionne le deuxième modèle de détection et le troisième modèle de détection dans un cas où le mouvement du sujet est inférieur à la première référence,
détecte les points caractéristiques à partir de la première image de caméra en utilisant le troisième modèle de détection,
détecte les points caractéristiques à partir de la deuxième image de caméra en utilisant le deuxième modèle de détection,
compare une précision de détection des points caractéristiques détectés en utilisant le troisième modèle de détection avec une précision de détection des points caractéristiques détectés en utilisant le deuxième modèle de détection,
spécifie la plage d'imagerie sur la base des points caractéristiques détectés en utilisant le troisième modèle de détection dans un cas où la précision de détection des points caractéristiques détectés en utilisant le troisième modèle de détection est plus élevée, et
spécifie la plage d'imagerie sur la base des points caractéristiques détectés en utilisant le deuxième modèle de détection dans un cas où la précision de détection des points caractéristiques détectés en utilisant le deuxième modèle de détection est plus élevée.

15. Appareil de traitement d'informations selon la revendication 14,

dans lequel le processeur
détermine la précision de détection des points caractéristiques détectés en utilisant le premier modèle de détection dans un cas où le mouvement est égal ou supérieur à la première référence, la précision de détection des points caractéristiques détectés en utilisant le troisième modèle de détection, ou la précision de détection des points caractéristiques détectés en utilisant le deuxième modèle de détection,
spécifie, dans un cas où la précision de détection est égale ou supérieure à une deuxième référence, la plage d'imagerie sur la base des points caractéristiques détectés en utilisant le premier modèle de détection dans un cas où le mouvement est égal ou supérieur à la première référence, des points caractéristiques détectés en utilisant le troisième modèle de détection, ou des points caractéristiques détectés en utilisant le deuxième modèle de détection, et émet une alerte dans un cas où la précision de détection est inférieure à la deuxième référence.

16. Appareil de traitement d'informations selon l'une quelconque des revendications 1 à 15, dans lequel le processeur dérive une plage de mouvement de la table d'examen sur la base de la plage d'imagerie.

17. Appareil de traitement d'informations selon la revendication 16, dans lequel le processeur affiche, sur un affichage, une image de corps humain imitant un corps humain et dessine, sur l'image de corps humain, une ligne de début de mouvement et une ligne de fin de mouvement de la table d'examen sur la base de la plage de mouvement de la table d'examen.

18. Appareil de traitement d'informations selon l'une quelconque des revendications 1 à 17, dans lequel la plage d'imagerie est une plage d'imagerie dans un cas de capture d'une image de positionnement acquise avant que l'imagerie principale du sujet ne soit effectuée.

19. Procédé de traitement d'informations comprenant :

via un ordinateur,
acquérir au moins l'une d'une première image de caméra générée en capturant une image en mouvement d'un sujet sur une table d'examen via une première caméra ou
d'une deuxième image de caméra générée en capturant une image en mouvement du sujet via une deuxième caméra ayant une sensibilité d'imagerie supérieure à celle de la première caméra ;
sélectionner au moins un modèle de détection parmi une pluralité de modèles de détection incluant un premier modèle de détection construit pour détecter une pluralité de points caractéristiques sur le sujet inclus dans la première image de caméra, et un deuxième modèle de détection construit pour détecter la pluralité de points caractéristiques sur le sujet inclus dans la deuxième image de caméra ;
détecter la pluralité de points caractéristiques sur le sujet inclus dans la première image de caméra ou dans la deuxième image de caméra en utilisant le modèle de détection sélectionné ; et
spécifier une plage d'imagerie du sujet sur la base de la pluralité de points caractéristiques.

20. Support de stockage lisible par ordinateur qui stocke un programme de traitement d'informations amenant un ordinateur à exécuter :

une procédure d'acquisition d'au moins l'une d'une première image de caméra générée en capturant une image en mouvement d'un sujet sur une table d'examen via une première caméra ou d'une deuxième image de caméra générée en capturant une image en mouvement du sujet via une deuxième caméra ayant une sensibilité d'imagerie supérieure à celle de la première caméra ;
une procédure de sélection d'au moins un modèle de détection parmi une pluralité de modèles de détection incluant un premier modèle de détection construit pour détecter une pluralité de points caractéristiques sur le sujet inclus dans la première image de caméra, et un deuxième modèle de détection construit pour détecter la pluralité de points caractéristiques sur le sujet inclus dans la deuxième image de caméra ;
une procédure de détection de la pluralité de points caractéristiques sur le sujet inclus dans la première image de caméra ou dans la deuxième image de caméra en utilisant le modèle de détection sélectionné ; et
une procédure de spécification d'une plage d'imagerie du sujet sur la base de la pluralité de points caractéristiques.

FIG. 1

# FIG. 2

# FIG. 3

# FIG. 4

# FIG. 5

10

## INFORMATION PROCESSING APPARATUS

| IMAGING CONTROLLER | 20 |

| CAMERA CONTROLLER | 21 |

SELECTION UNIT — 22

| FIRST DETECTION MODEL | 22A |

| SECOND DETECTION MODEL | 22B |

| FEATURE POINT DETECTION UNIT | 23 |

| IMAGING RANGE SPECIFYING UNIT | 24 |

# FIG. 6

# FIG. 7

# FIG. 8

```
                    ┌─────────────┐
                    │   START     │
                    └──────┬──────┘
                           │           ST1
                    ┌──────▼──────────────┐
                    │ DETERMINE LIGHTNESS │
                    └──────┬──────────────┘
                           │           ST2
                    ┌──────▼──────────────┐
                    │ SELECT DETECTION MODEL │
                    └──────┬──────────────┘
                           │           ST3
                    ┌──────▼──────────────┐
                    │ ACQUIRE CAMERA IMAGES │
                    └──────┬──────────────┘
                           │           ST4
                    ┌──────▼──────────────┐
                    │ DETECT FEATURE POINTS │
                    └──────┬──────────────┘
                           │           ST5
                      ╱────▼──────────╲       LOW
                     ╱ DETERMINE DETECTION╲──────────┐
                     ╲    ACCURACY        ╱          │
                      ╲──────┬───────────╱           │
                           │ HIGH    ST6         ST8 │
                    ┌──────▼──────────────┐   ┌──────▼──────┐
                    │ SPECIFY IMAGING RANGE│   │ PERFORM ALERT│
                    └──────┬──────────────┘   │  DISPLAY    │
                           │           ST7    └──────┬──────┘
                    ┌──────▼──────────────┐          │
                    │    DRAW LINE        │          │
                    └──────┬──────────────┘          │
                           │           ST9           │
              NO      ╱────▼──────────╲              │
         ┌───────────╱ IS INSTRUCTION TO START╲      │
         │           ╲ SCOUT IMAGING ISSUED?  ╱      │
         │            ╲──────┬───────────────╱       │
         │                 │ YES      ST10           │
         │          ┌──────▼──────────────┐          │
         │          │ STOP IMAGING VIA CAMERA│        │
         │          └──────┬──────────────┘          │
         │                 │◄───────────────────────┘
         │          ┌──────▼──────┐
         │          │    END      │
         │          └─────────────┘
```

# FIG. 9

START

ST21

ACQUIRE RGB CAMERA
IMAGE

ST22

SELECT FIRST DETECTION
MODEL

ST23

DETECT FEATURE POINTS

ST24

DETERMINE LIGHTNESS — BRIGHT

DARK ST25

SELECT SECOND DETECTION
MODEL

ST26

DETECT FEATURE POINTS

ST27

DETERMINE DETECTION
ACCURACY — LOW

HIGH ST28          ST30

SPECIFY IMAGING RANGE    PERFORM ALERT
DISPLAY

ST29

DRAW LINE

ST31

NO — IS INSTRUCTION TO START
SCOUT IMAGING ISSUED?

YES ST32

STOP IMAGING VIA CAMERA

END

# FIG. 10

INFORMATION PROCESSING APPARATUS — 10A

| | |
|---|---|
| IMAGING CONTROLLER | ~20 |
| CAMERA CONTROLLER | ~21 |

SELECTION UNIT — 22

- FIRST DETECTION MODEL — 22A
- SECOND DETECTION MODEL — 22B
- THIRD DETECTION MODEL — 22C
- FOURTH DETECTION MODEL — 22D

FEATURE POINT DETECTION UNIT — 23

IMAGING RANGE SPECIFYING UNIT — 24

# FIG. 11

```
        ┌─────────────┐
        │    START    │
        └─────────────┘
               │            ST41
               ▼
   ┌───────────────────────────┐
   │   DETERMINE LIGHTNESS     │
   │    AND IMAGING PART       │
   └───────────────────────────┘
               │            ST42
               ▼
   ┌───────────────────────────┐
   │   SELECT DETECTION MODEL  │
   └───────────────────────────┘
               │            ST43
               ▼
   ┌───────────────────────────┐
   │    ACQUIRE CAMERA IMAGES  │
   └───────────────────────────┘
               │            ST44
               ▼
   ┌───────────────────────────┐
   │    DETECT FEATURE POINTS  │
   └───────────────────────────┘
               │            ST45
               ▼
   ⬡ DETERMINE DETECTION ⬡ ───── LOW
   ⬡      ACCURACY       ⬡
               │ HIGH      ST46              ST48
               ▼                    ┌─────────────────┐
   ┌───────────────────────────┐    │ PERFORM ALERT   │
   │   SPECIFY IMAGING RANGE   │    │    DISPLAY      │
   └───────────────────────────┘    └─────────────────┘
               │            ST47
               ▼
   ┌───────────────────────────┐
   │        DRAW LINE          │
   └───────────────────────────┘
               │            ST49
               ▼
   NO ⬡ IS INSTRUCTION TO START ⬡
      ⬡ SCOUT IMAGING ISSUED?   ⬡
               │ YES       ST50
               ▼
   ┌───────────────────────────┐
   │  STOP IMAGING VIA CAMERA  │
   └───────────────────────────┘
               │
               ▼
        ┌─────────────┐
        │     END     │
        └─────────────┘
```

# FIG. 12

```
                    ┌──────────┐
                    │  START   │
                    └──────────┘
   ③ ──────────────────────┐
                           │         ST61
              ┌────────────▼────────────┐
              │  ACQUIRE CAMERA IMAGES   │
              └────────────┬────────────┘
                           │         ST62
              ┌────────────▼────────────┐
              │  SELECT FIRST DETECTION  │
              │         MODEL            │
              └────────────┬────────────┘
                           │         ST63
              ┌────────────▼────────────┐
              │   DETECT FEATURE POINTS  │
              └────────────┬────────────┘
                           │         ST64
          ╱────────────────▼────────────────╲   LOW
          ⟨    DETERMINE DETECTION           ⟩─────▶ ①
          ╲        ACCURACY                  ╱
           ╲──────────────┬─────────────────╱
                     HIGH │         ST65
              ┌───────────▼─────────────┐
              │  SELECT THIRD DETECTION  │
              │         MODEL            │
              └───────────┬─────────────┘
                          │          ST66
              ┌───────────▼─────────────┐
              │   DETECT FEATURE POINTS  │
              └───────────┬─────────────┘
                          │          ST67
          ╱───────────────▼────────────────╲   HIGH
          ⟨    DETERMINE DETECTION          ⟩─────▶ ①
          ╲        ACCURACY                 ╱
           ╲──────────────┬────────────────╱
                      LOW │         ST68
              ┌───────────▼─────────────┐
              │  SELECT FOURTH DETECTION │
              │         MODEL            │
              └───────────┬─────────────┘
                          │
                          ▼
                         ②
```

# FIG. 13

② → ST69

DETECT FEATURE POINTS

↓ ST70

DETERMINE DETECTION ACCURACY — LOW →

HIGH

① →

ST71

SPECIFY IMAGING RANGE

ST73

PERFORM ALERT DISPLAY

↓ ST72

DRAW LINE

↓ ST74

NO ← ③   IS INSTRUCTION TO START SCOUT IMAGING ISSUED?

YES   ST75

STOP IMAGING VIA CAMERA

↓

END

## FIG. 14

10B

INFORMATION PROCESSING APPARATUS

IMAGING CONTROLLER —20

CAMERA CONTROLLER —21

SELECTION UNIT —22

FIRST DETECTION MODEL —22A

SECOND DETECTION MODEL —22B

THIRD DETECTION MODEL —22C

FOURTH DETECTION MODEL —22D

FEATURE POINT DETECTION UNIT —23

IMAGING RANGE SPECIFYING UNIT —24

MOVEMENT DETECTION UNIT —25

# FIG. 15

START

3 → ST81

ACQUIRE CAMERA IMAGES

ST82

SELECT FIRST DETECTION MODEL

ST83

DETECT FEATURE POINTS

ST84

DETECT MOVEMENT

ST85

IS MOVEMENT LARGE? — YES → 2

NO ST86

SELECT THIRD DETECTION MODEL

ST87

DETECT FEATURE POINTS

ST88

SELECT FOURTH DETECTION MODEL

ST89

DETECT FEATURE POINTS

1

# FIG. 16

① 

ST90
⟨ COMPARE DETECTION ACCURACY ⟩ —— R3 < R4

R3 ≥ R4

ST91
USE FEATURE POINTS DETECTED BY USING THIRD DETECTION MODEL

ST92
USE FEATURE POINTS DETECTED BY USING FOURTH DETECTION MODEL

②

ST93
⟨ DETERMINE DETECTION ACCURACY ⟩ —— LOW

HIGH

ST94
SPECIFY IMAGING RANGE

ST96
PERFORM ALERT DISPLAY

ST95
DRAW LINE

ST97
NO —— ⟨ IS INSTRUCTION TO START SCOUT IMAGING ⟩ ③

YES ST98
STOP IMAGING VIA CAMERA

END

**EP 4 548 853 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021079013 A
- US 2017224298 A1
- CN 104257396 A